(19) Europäisches Patentamt
European Patent Office
Office européen des brevets

(11) EP 4 357 384 A1

(12) EUROPEAN PATENT APPLICATION
published in accordance with Art. 153(4) EPC

(43) Date of publication:
**24.04.2024 Bulletin 2024/17**

(21) Application number: **21945739.7**

(22) Date of filing: **20.10.2021**

(51) International Patent Classification (IPC):
***C08G 61/12*** *(2006.01)* ***H01L 51/30*** *(2006.01)*
***H01L 51/46*** *(2006.01)* ***H01L 51/54*** *(2006.01)*

(52) Cooperative Patent Classification (CPC):
**C08G 61/02; C08G 61/12; H05K 9/00; H10K 50/00; H10K 85/00; H10N 10/856;** Y02E 10/549

(86) International application number:
**PCT/CN2021/124880**

(87) International publication number:
**WO 2022/262159 (22.12.2022 Gazette 2022/51)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **18.06.2021 CN 202110679959**

(71) Applicant: **South China University of Technology Guangzhou, Guangdong 510640 (CN)**

(72) Inventors:
- **HUANG, Fei**
  **Guangzhou, Guangdong 510640 (CN)**
- **TANG, Haoran**
  **Guangzhou, Guangdong 510640 (CN)**

(74) Representative: **Schulz Junghans Patentanwälte PartGmbB Großbeerenstraße 71 10963 Berlin (DE)**

# (54) N-TYPE CONJUGATED POLYMER, PREPARATION METHOD THEREFOR, AND APPLICATION THEREOF

(57) The present invention relates to the technical field of n-type semiconductor materials. Disclosed are an n-type conjugated polymer, a preparation method therefor, and a use thereof. In the method, using a solvent as a reaction medium, a reaction monomer is reacted under the action of a substance having oxidability to obtain an n-type conjugated polymer. The n-type conjugated polymer comprises one or more polymerization units, and the polymerization unit are of a structure of formula (I) and/or a structure of formula (II), and/or in an enol-type transformation form corresponding thereto. According to the present invention, an aromatic diketone substance having active methylene is a raw material, and is subjected to a direct polymerization reaction by means of the substance having the oxidability. The reaction does not require a noble metal for catalysis, and the n-type conjugated polymer is not sensitive to the reaction atmosphere, has a simple process and low costs, and is suitable for commercial applications. The n-type conjugated polymer of the present invention has excellent electron transport capability, higher conductivity, and better electromagnetic wave shielding effect. The n-type conjugated polymer of the present invention is applied to an organic optoelectronic device, and can achieve an excellent photoelectric effect.

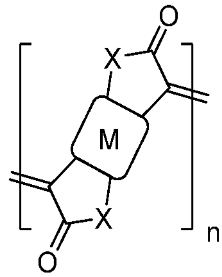
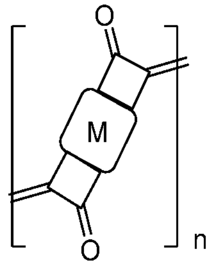

(I) (II)

EP 4 357 384 A1

## Description

### Technical Field

The present invention belongs to an n-type organic semiconductor materials technical field, and specifically, relates to an n-type conjugated polymer, a preparation method and a use thereof.

### Background

A structure of a conjugated polymer contains a conjugated system composed of delocalized $\pi$-electrons. Therefore, the conjugated polymer exhibits special optical and electrical properties, and has drawn widespread attention from scientists. Photoelectric materials and semiconductor materials applied to devices have not only electronic properties of metals or semiconductors, but also the characteristics of low costs, light weight, low-temperature processing, and easy large-area preparation. They meet the requirements of large-scale industrial production and large-area promotion, and have broad commercial prospects. Currently, the conjugated polymer has achieved a series of impressive results in the fields of organic photovoltaic (OPV) cells, organic light emitting diodes (OLED), organic field effect transistors (OFET), and organic thermoelectrics (OTE).

A high-performance organic electron device usually needs p-type and n-type semiconductor materials at the same time during operation. PEDOT:PSS, as one of the p-type materials currently commercially applied, has achieved great success in semiconductors due to its characteristics of high conductivity and printability.

However, compared with the mature p-type material, an n-type organic material, especially an organic n-type conjugated polymer with high conductivity, lacks a corresponding synthesis strategy. Due to the limitation of factors, such as low electron mobility, poor air stability, and a need for a long insulating alkyl chain for allowing solution processing, of the n-type organic semiconductor material, development of an n-type organic semiconductor material that has high conductivity, simple synthesis process, and low costs and that is applicable to solution processing is a hotspot in the current research.

A 3,7-dihydrobenzo[1,2-b:4,5-b']difuran-2,6-dione molecule contains two strong electron-withdrawing functional groups, which can effectively reduce the lowest unoccupied molecular orbital (LUMO) of a polymer. Therefore, the electron mobility of the polymer is improved, and the stability of n-type doping of the polymer is ensured. There are many reports on polymers based on 3,7-dihydrobenzo[1,2-b:4,5-b']difuran-2,6-dione. For example, a document (Toward High Performance n-Type Thermoelectric Materials by Rational Modification of BDPPV Backbones. J. Am. Chem. Soc. 2015, 137, 6979) reports a benzodifurandione-based on polymer BDPPV whose highest conductivity of 14 S/cm can be achieve by optimizing a dopant. A document (High Conductivity and Electron-Transfer Validation in an n-Type Fluoride-Anion-Doped Polymer for Thermoelectrics in Air. Adv. Mater. 2017, 29, 1606928) reports an n-type thermoelectric material that has good air stability and is obtained by doping a benzodifurandione-based polymer with tetrabutylammonium fluoride. In addition, a document (Rigid Coplanar Polymers for Stable n-Type Polymer Thermoelectrics. Angew. Chem. Int. Ed. 2019, 58, 11390) reports a benzodifurandione-based polymer LPPV that has air stability and high conductivity and is obtained through acid-catalyzed aldol condensation reaction polymerization without using a noble metal catalyst.

However, due to the excellent planarity of 3,7-dihydrobenzo[1,2-b:4,5-b']difuran-2,6-dione, a long alkyl chain needs to be introduced on a polymerization unit of current most polymers based on this unit to ensure the solubility of the polymers in organic solvents. Moreover, an additional dopant (such as N-DMBI) is used for doping to achieve high conductivity. The polymers cannot keep stable under air atmosphere for a long term.

For diketone fused ring compounds, a document (Synthese, Charakterisierung und Halbleitereigenschaften eines polymeren Indopheninhomologen. Macro. Chem. Phys. 1975, 176, 333) discloses a p-type quinoid polymer with conductivity that can be formed through dehydration polymerization under the condition of sulfuric acid catalysis. The patent application WO 2014/071524 discloses a polymer containing a fused ring structure, and introduces its application as an organic semiconductor in OFET. The patent application US 2019/0048015 A1 discloses a class of near-infrared organic semiconductor polymers without noble metal catalysis.

In addition, the patent application CN108699073 discloses a semiconductor polymer and a synthesis method thereof. A structure of the semiconductor polymer is

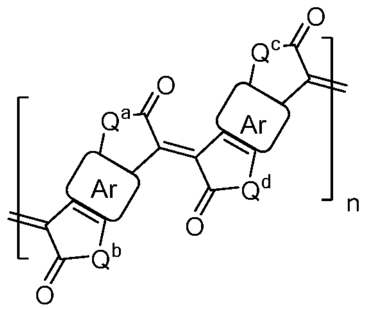

. However, during preparation of the polymer, tetraketone A

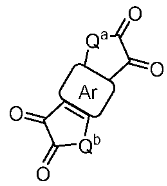

is employed as a raw material, which is an intermediate after oxidation reaction. Synthesis of this intermediate needs additional processing costs, so the synthesis route is uneconomical, and synthesis is difficult to commercialize. Moreover, actually for some tetraketone A structures such as

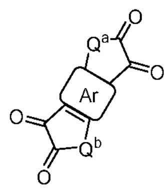

, a preparation method is not disclosed in the prior art, so a polymer preparation method suitable for commercialization is difficult to obtain from the disclosed documents.

The foregoing disclosed polymers do not involve any n-type conductive properties, or the relevant data are not ideal. All the disclosed polymer structures contain an alkyl side chain, and there is no report on a polymer structure that allows solution processing in the absence of an alkyl chain and a preparation method.

Based on the above, development of a class of n-type highly conductive conjugated polymers that have simple structures, simple synthesis process, and low costs, are applicable to solution processing, are directly synthesized by a preparation method suitable for commercialization, and are applied in the organic photoelectric field to achieve an ideal photoelectric effect is a problem to be solved urgently.

## Summary

In order to overcome the shortcomings and deficiencies in the prior art, an objective of the present invention is to provide an n-type conjugated polymer and a preparation method thereof. In the method of the present invention, an aromatic diketone substance having active methylene is employed as a raw material, and an n-type conjugated polymer is directly prepared through a polymerization reaction in the presence of an oxidant. The reaction does not require noble metal catalysis and is not sensitive to the reaction atmosphere, the process is simple, and the cost is low, so the preparation method is suitable for commercial application. The n-type conjugated polymer prepared by the method of the present invention has good solubility in a general organic solvent, thus allowing solution processing. Moreover, when applied in an organic photoelectric device, the n-type conjugated polymer can achieve an excellent photoelectric effect.

Another objective of the present invention is to provide a use of the n-type conjugated polymer prepared by the foregoing method. The n-type conjugated polymer is applied in an organic photoelectric device. Specifically, the n-type conjugated polymer of the present invention is applied in an organic photovoltaic cell as an electron transport layer, or as a thermoelectric material, or as an electromagnetic shielding material.

Objectives of the present invention are realized by the following technical solutions.

An n-type conjugated polymer is a homopolymer or copolymer. The n-type conjugated polymer includes one

or more polymerization units. The polymerization unit is a structure represented by formula I and/or a structure represented by formula II, and/or a corresponding enol-type transformation form thereof:

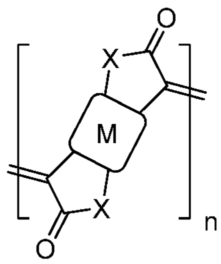

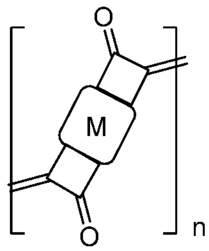

formula I formula II;

Further, a structure of the n-type conjugated polymer is specifically formula I, formula II, a structure composed of formula I and formula II, a structure composed of formula I and formula I, a structure composed of formula II and formula II, an enol-type transformation form corresponding to formula I, an enol-type transformation form corresponding to formula II, a structure composed of formula I and the corresponding enol-type transformation form thereof, a structure composed of formula II and the corresponding enol-type transformation form thereof, or a structure composed of formula I, formula II, and the corresponding enol-type transformation forms. In the structure composed of formula I and formula I, formula I is different from formula I. In the structure composed of formula II and formula II, formula II is different from formula II. The structure composed of formula I and formula II contains one or more formulae I, and contains one or more formulae II, formulae I are the same or different, and formulae II are the same or different.

In formula I, X is independently selected from O, S, Se, Te or N-$R_1$;

the enol-type transformation form corresponding to formula I is selected from one or more of the following structures:

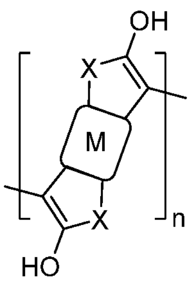

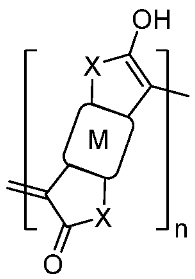

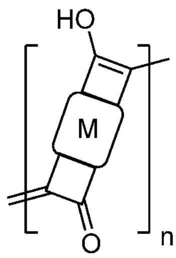

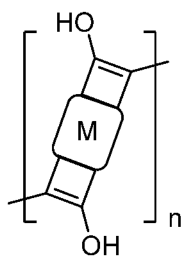

;

and $R_1$ is selected from one or more of a hydrogen atom, alkyl, alkylene (R'-$C_{n'}H_{2n'}$, R' is a substituent group, and $C_{n'}H_{2n'}$ is alkylene), an alkyl derivative, and an alkylene derivative;

the alkyl derivative refers to that one or more carbons on alkyl are substituted with one or more of an oxygen atom, amino, sulfonyl, carbonyl, aryl, alkenyl, alkynyl, ester, cyano, and nitro;
and/or

one or more hydrogens on the alkyl derivative are substituted with one or more of a halogen, hydroxyl, amino, carboxyl, cyano, nitro, aryl, alkenyl, and alkyne;

n is a positive integer;

M is a conjugated part in the structure of the n-type conjugated polymer, and a structure of M is selected from one of an aromatic ring, a heteroaromatic ring, a fused aromatic ring, and a fused heteroaromatic ring; and

the aromatic ring refers to a ring structure that has a conjugated planar ring system and in which a bond between atoms is covered by a delocalized $\pi$-electron cloud. For example, the aromatic ring is a benzene ring or a derivative thereof.

The heteroaromatic ring refers to a ring structure that has a conjugated planar ring system and in which a bond between atoms is covered by a delocalized π-electron cloud and atoms forming the ring further contain at least one heteroatom (such N, O or S) in addition to a carbon atom. For example, the heteroaromatic ring is thiophene, furan or pyrrole.

The fused aromatic ring refers to a structure formed by fusing (that is, sharing a ring edge) two or more aromatic rings that have a conjugated planar ring system and in which a bond between atoms is covered by a delocalized π-electron cloud. For example, the fused aromatic ring is naphthalene, anthracene, or a derivative thereof.

The fused heteroaromatic ring refers to a structure formed by fusing two or more aromatic rings that have a conjugated planar ring system and in which a bond between atoms is covered by a delocalized π-electron cloud, and furthermore, constituent atoms in at least one ring further contain at least one heteroatom (such N, O or S) in addition to a carbon atom. For example, the fused heteroaromatic ring is quinoline, indole, or a derivative thereof.

In the present invention, the n-type highly conductive conjugated polymer has a plurality of resonance forms. In a case that M is a benzene ring structure, the n-type highly conductive conjugated polymer includes, but is not limited to, the following resonance forms:

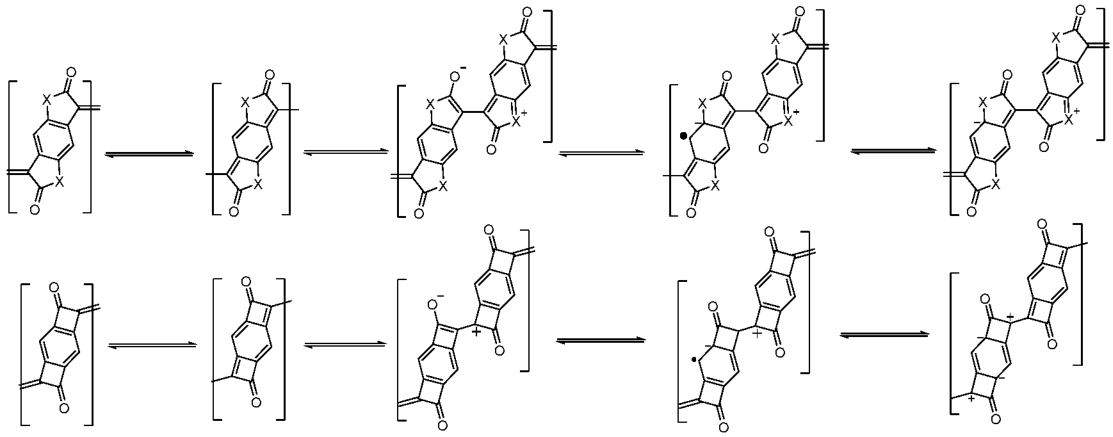

; and

moreover, due to the presence of the enol-type transformation form, in a case that M is a benzene ring structure, the n-type highly conductive conjugated polymer includes, but is not limited to, the following resonance forms:

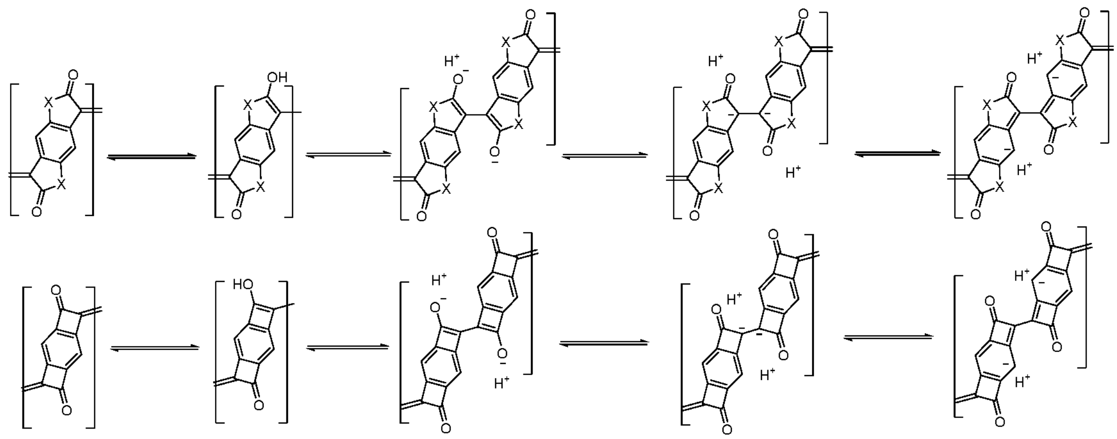

.

Further, the different resonance forms may form the following supramolecules through self-assembly under the intermolecular interaction:

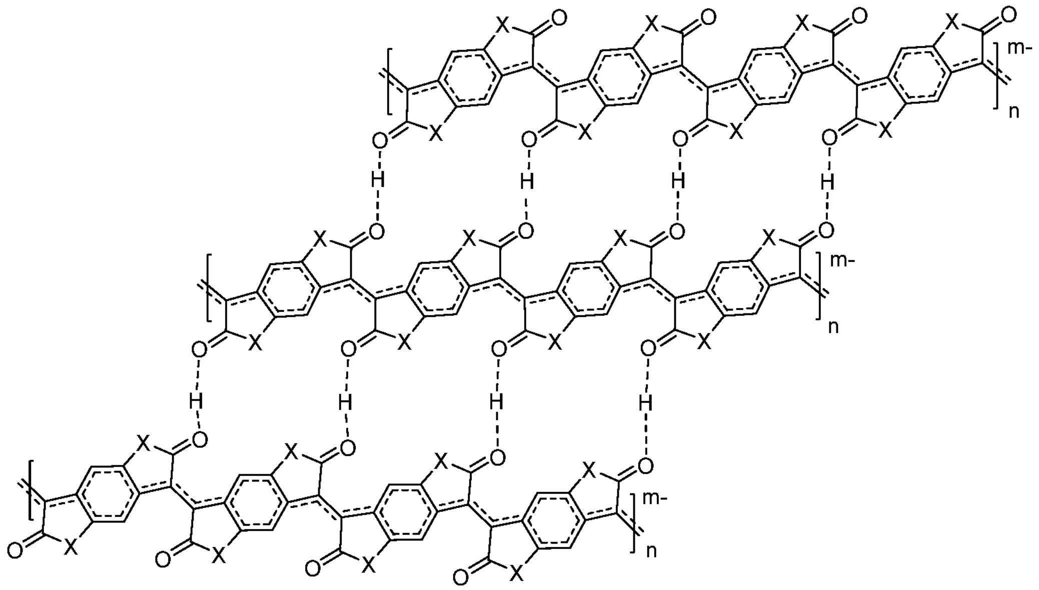

It should be noted that in a case that X is an oxygen atom, and M is a benzene ring structure, the prepared compound further has the following tautomers:

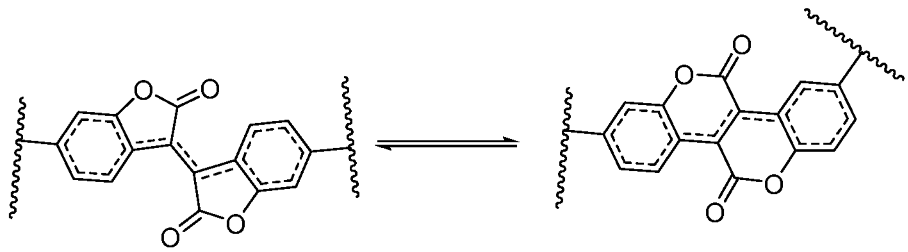

,

and the break line indicates a chain segment of the conjugated polymer.

For the purpose of convenience, the present invention is described with reference to the first resonance form.

Further, the structure of M is selected from the following structures:

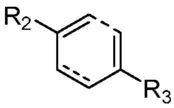 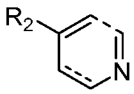 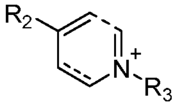 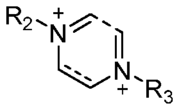

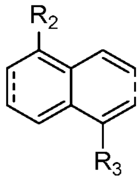 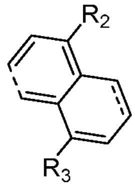 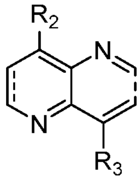 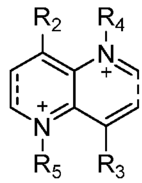

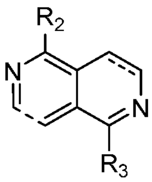 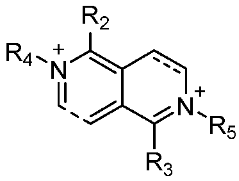 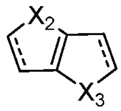 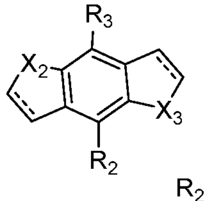

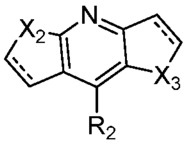 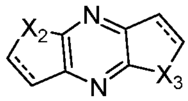 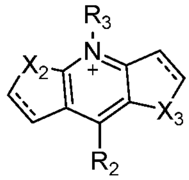 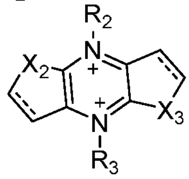

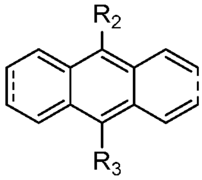 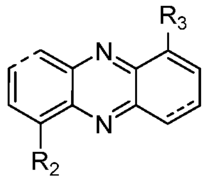 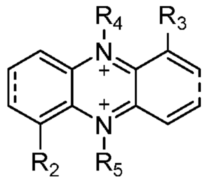 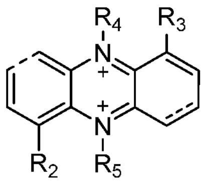

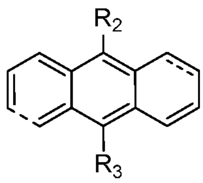 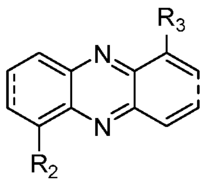 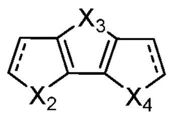 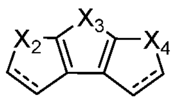 ;

where, $X_2$ to $X_4$ are independently selected from O, S, Se, Te or N-$R_1$;

$R_2$ to $R_5$ are independently selected from one or more of a hydrogen atom, hydroxyl, nitro, a halogen, cyano, nitro, alkyl, and an alkyl derivative;

one or more carbons on the alkyl derivative are substituted with one or more of an oxygen atom, amino, sulfonyl, carbonyl, aryl, alkenyl, alkynyl, ester, cyano, and nitro;
and/or

one or more hydrogens on the alkyl derivative are substituted with one or more of a halogen, hydroxyl, amino, carboxyl, cyano, nitro, aryl, alkenyl, and alkyne.

It should be noted that in the foregoing optional objects of the structure of M, a dotted line (---) in the aromatic ring indicates mutual fusion of the aromatic ring and an adjacent five-membered ring or four-membered ring at this place, namely, a shared ring edge. For example, in a case that the structure of M is represented as

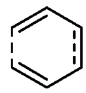

, an actual structure of the n-type conjugated polymer is:

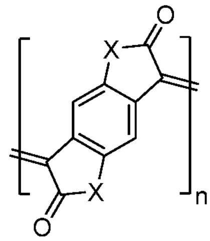

or

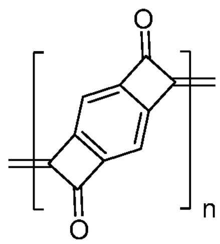

,

or a corresponding enol-type transformation form thereof.

Further, the n-type conjugated polymer is a homopolymer or copolymer.

In a case that the n-type conjugated polymer is a homopolymer, the structure of the n-type conjugated polymer is selected from one of the following structures or corresponding enol-type transformation forms thereof:

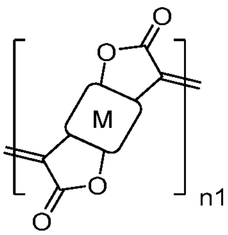
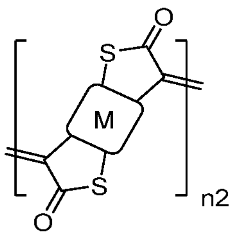
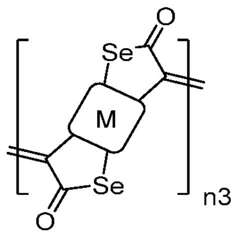
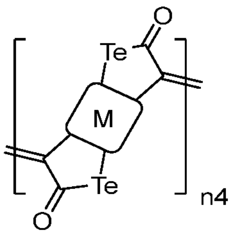
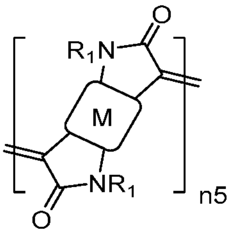
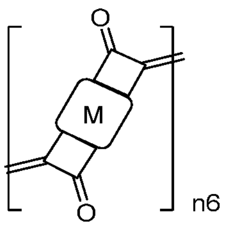

.

In a case that the n-type conjugated polymer is a copolymer, the n-type conjugated polymer includes at least two kinds of polymerization units;

a structure of each polymerization unit is independently selected from the following structures:

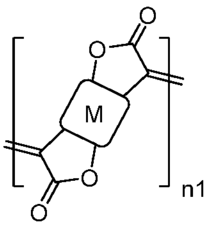 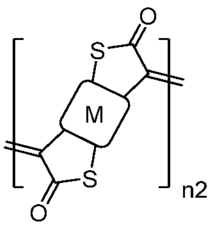 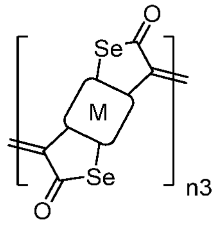

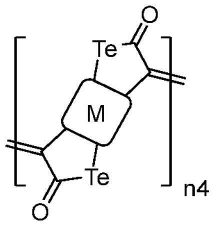 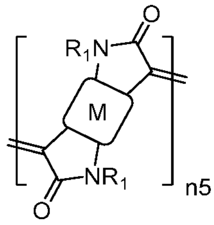 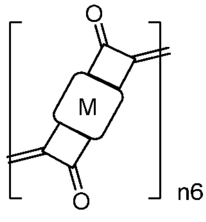 ,

or corresponding enol-type transformation forms thereof,

in the structure,

$R_1$ is selected from one or more of a hydrogen atom, alkyl, and an alkyl derivative;

one or more carbons on the alkyl derivative are substituted with one or more of an oxygen atom, amino, sulfonyl, carbonyl, aryl, alkenyl, alkynyl, ester, cyano, and nitro;
and/or

one or more hydrogens on the alkyl derivative are substituted with one or more of a halogen, hydroxyl, amino, carboxyl, cyano, nitro, aryl, alkenyl, and alkyne;

n1 to n6 are independently a positive integer; and

M is a conjugated part in the structure of the n-type conjugated polymer, and a structure of M is selected from one of an aromatic ring, a heteroaromatic ring, a fused aromatic ring, and a fused heteroaromatic ring.

$R_1$ and M in the foregoing structure have the same definitions as those in formula I or formula II.

A preparation method for an n-type conjugated polymer includes the following steps:
using a solvent as a reaction medium, a reaction monomer reacting under the action of a substance having oxidability to obtain an n-type conjugated polymer.

The reaction monomer is

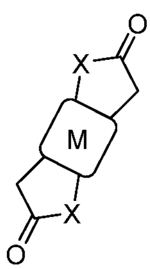

and/or

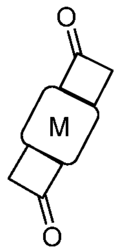

, and/or a corresponding enol-type transformation form thereof.

The corresponding enol-type transformation form is selected from one or more of the following structures:

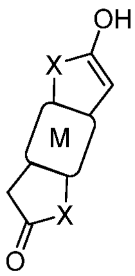

,

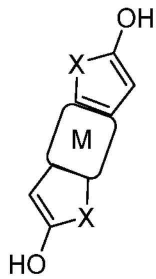

,

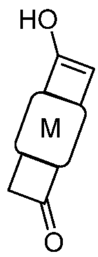

,

and

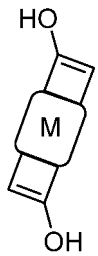

.

M and X in the structures have the same definitions as those in the foregoing n-type conjugated polymer.

In a case that the n-type conjugated polymer is a homopolymer, the reaction monomers are compounds with the same structure.

In a case that the n-type conjugated polymer is a copolymer, the reaction monomers are compounds with different structures. That is, the reaction monomers are more than two kinds of compounds represented by formula I' or more than two kinds of compounds represented by formula II', or corresponding enol-type transformation forms thereof; or the reaction monomers are a compound represented by formula I' and a compound represented by formula II', and/or corresponding enol-type transformation forms thereof.

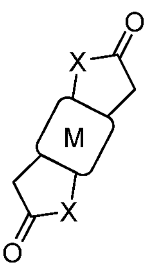

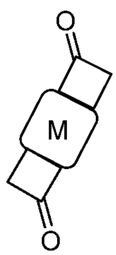

formula I′ formula II′.

In a case that the n-type conjugated polymer is a copolymer, the preparation method for the n-type conjugated

polymer can also comprise the following steps:

1) reacting different reaction monomers in the solvent under the action of the substance having oxidability to obtain different polymerization units; and

2) using the solvent as a reaction medium, further performing copolymerization on the different polymerization units obtained in step 1) under the action of the substance having oxidability to obtain the n-type conjugated polymer.

The different polymerization units are preferably more than two of the following structures, and/or corresponding enol-type transformation forms thereof:

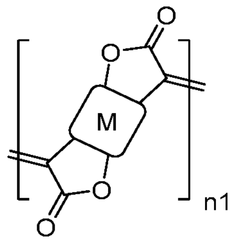
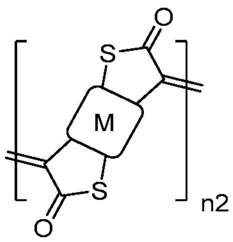
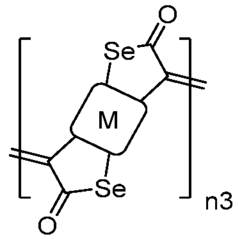
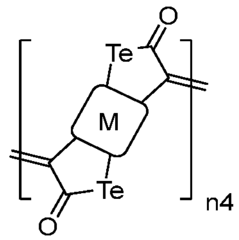
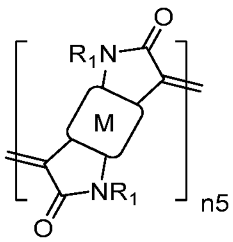
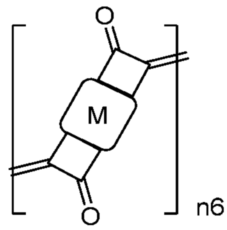

In a case that the n-type conjugated polymer is a copolymer, the copolymer may be a bipolymer or multipolymer. Different polymerization units may be polymerized with each other to form a new long-chain copolymer. A structure of the copolymer may be, but is not limited to, the following structures:

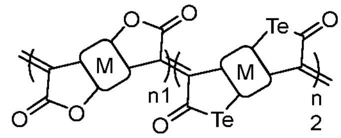
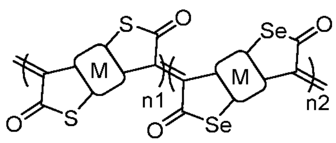
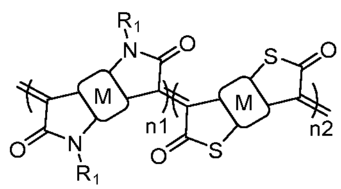
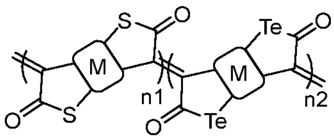
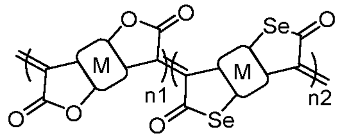
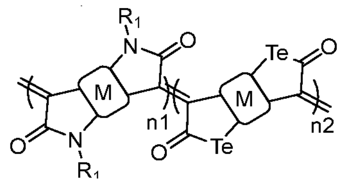
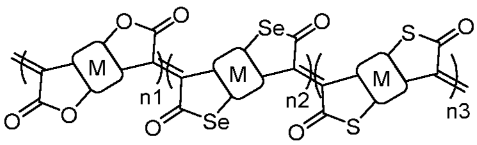
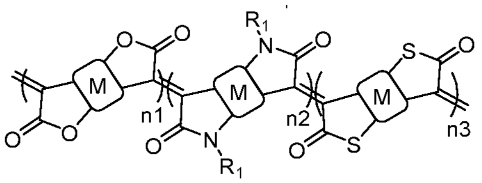

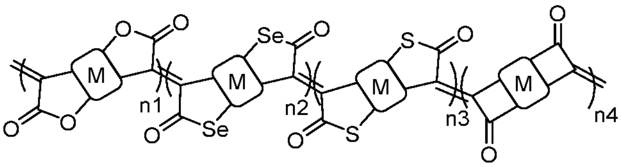

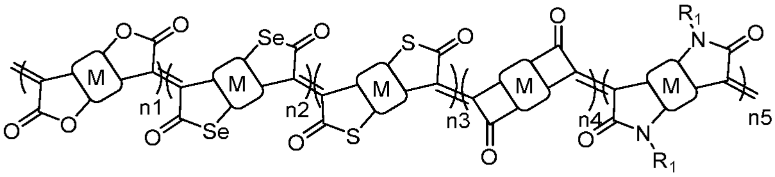

The substance having oxidability is selected from a mixture of one or more of organic substances having oxidability and inorganic substance having oxidability.

Further, the substance having oxidability is more than one of oxygen, a peroxide, a metal halide, a persulfate, a perborate, a hypohalite, a chlorite, a bromite, a quinone compound, and a perbenzoate compound.

Specifically, the foregoing substance having oxidability may be, but is not limited to, more than one of oxygen, hydrogen peroxide, sodium peroxide, potassium peroxide, calcium peroxide, zinc peroxide, copper peroxide, iron nitrate, zinc nitrate, nickel nitrate, aluminium nitrate, magnesium nitrate, ammonium nitrate, iron fluoride, iron chloride, iron bromide, iron iodide, sodium perchlorate, potassium perchlorate, sodium perbromate, potassium perbromate, sodium periodate, potassium periodate, potassium perchlorate, sodium perchlorate, potassium perbromate, sodium perbromate, magnesium perchlorate, sodium persulfate, potassium persulfate, magnesium persulfate, zinc persulfate, iron persulfate, copper persulfate, calcium persulfate, potassium perborate, zinc perborate, magnesium perborate, calcium perborate, sodium hypofluorite, potassium hypofluorite, sodium hypochlorite, potassium hypochlorite, iron hypochlorite, copper hypochlorite, sodium hypobromite, potassium hypobromite, sodium hypoiodite, potassium hypoiodite, sodium chlorite, potassium chlorite, iron chlorite, sodium bromite, potassium bromite, sodium iodate, potassium iodate, benzoquinone and a derivative thereof, naphthoquinone and a derivative thereof, anthraquinone and a derivative thereof, phenanthrenequinone and a derivative thereof, and perbenzoic acid and a derivative thereof.

The solvent is selected from a solvent 1, a solvent 2, or a mixture of the solvent 1 and the solvent 2;

the solvent 1 is selected from a mixture of one or more of water, a nitrile solvent, an aromatic solvent, an alicyclic hydrocarbon solvent, an alicyclic hydrocarbon solvent, a halogenated hydrocarbon solvent, an alcohol solvent, an ether solvent, an ester solvent, a sulfone solvent, a ketone solvent, an amide solvent; and

the solvent 2 is a deuterated solvent of the solvent 1.

Specifically, the foregoing solvent 1 is preferably a polar solvent, and may be, but is not limited to, more than one of tetrahydrofuran, methyltetrahydrofuran, dichloromethane, chloroform, ethyl acetate, propyl acetate, butyl acetate, ethyl propionate, propyl propionate, butyl propionate, methyl methacrylate, ethyl methacrylate, propyl methacrylate, toluene, xylene, trimethylbenzene, chlorobenzene, dichlorobenzene, trichlorobenzene, methanol, ethanol, propanol, ethylene glycol, isobutanol, propylene glycol, acetonitrile, formic acid, acetic acid, propionic acid, trifluoroacetic acid, dimethylformamide, dimethylacetamide, dimethyl sulfoxide, dimethylacetamide, acetone, butanone, cyclohexanone, methyl butanone, methyl ether, diethyl ether, propyl ether, pyridine, phenol, N-methylpyrrolidone, ethylene glycol monomethyl ether, triethylene glycol monomethyl ether, triethylamine, tetramethylethylenediamine, trioctylamine, aniline, and hexamethylphosphoramide.

Specifically, the foregoing solvent 2 is a deuterated solvent corresponding to the foregoing solvent 1. For example, the solvent 2 may be, but is not limited to, deuterated chloroform, deuterated chlorobenzene, deuterated ethanol, and the like.

Preferably, the solvent is selected from the solvent 1.

The foregoing n-type conjugated polymer is applied in an organic photoelectric device. The n-type conjugated polymer is applied in an electron transport layer. The electron transport layer includes the foregoing n-type conjugated polymer.

The n-type conjugated polymer is applied in a thermoelectric material. The thermoelectric material includes the foregoing n-type conjugated polymer.

The n-type conjugated polymer is applied in an electromagnetic shielding material. The electromagnetic shielding material includes the foregoing n-type conjugated polymer.

Relative to the prior art, the present invention has the following advantages and beneficial effects.

1. According to the present invention, the n-type conjugated polymer is directly prepared through a polymerization reaction of an aromatic diketone substance having active methylene that serves as a raw material in the presence of a substance having oxidability. The reaction does not require noble metal catalysis and is not sensitive to the reaction atmosphere, the process is simple, the cost is low, so the preparation method is suitable for large-scale commercial application. The prepared product has excellent solubility and thus is applicable to solution processing of an organic photoelectric device.

2. The n-type conjugated polymer of the present invention can be applied in an organic photovoltaic cell as an electron transport layer. Compared with the conventional electron transfer layer ZnO, the n-type conjugated polymer of the present invention has better electron transport capability and thus has better photoelectric conversion efficiency.

3. The n-type conjugated polymer of the present invention can be used as a thermoelectric material with highest conductivity of 1060 S/cm without using an additional dopant. A power factor exceeding 130 $\mu Wm^{-1}K^{-2}$ can be obtained when the n-type conjugated polymer of the present invention is applied in the thermoelectric material.

4. The n-type conjugated polymer of the present invention can be used as an electromagnetic shielding material that can shield more than 96% of electromagnetic waves at a thickness of 2 mm and function over a wide frequency range.

## Brief Description of Drawings

FIG. 1(a), FIG. 1(b), and FIG. 1(b) are absorption spectrograms of n-type conjugated polymers of Example 1, Example 6, and Example 7 in a solution or thin film state, respectively;

FIG. 2 is a J-V curve chart of an organic photovoltaic cell in which ZnO or an n-type conjugated polymer of Example 1 is employed as an electron transport layer of Test 2;

FIG. 3(a) is a thermal voltage difference change trend diagram of an n-type conjugated polymer of Example 2, and FIG. 3(b) is a fitted change trend diagram of the thermal voltage difference with temperature of the n-type conjugated polymer of Example 2;

FIG. 4 is a conductivity-Seebeck coefficient-power factor performance diagram of n-type conjugated polymers of Example 1 and Example 2; and

FIG. 5(a) is an electromagnetic shielding proportion diagram of an n-type conjugated polymer of Example 2 at different frequencies, and FIG. 5(b) is an electromagnetic shielding efficiency diagram of the n-type conjugated polymer of Example 2 at different frequencies.

## Detailed Description of the Embodiments

In order to describe the technical solutions of the present invention more clearly, the following examples are listed. Unless otherwise stated, raw materials, reactions, and post-processing methods in the examples are common raw materials on the market and technical means well-known to those skilled in the art.

3,7-dihydrobenzo[1,2-b:4,5-b']difuran-2,6-dione, serving as a raw material in the examples of the present invention, is prepared with reference to the document (A BDOPV-Based Donor-Acceptor Polymer for High-Performance n-Type and Oxygen-Doped Ambipolar Field-Effect Transistors. Adv. Mater. 2013, 25 (45), 6589). A substance having oxidability in the examples is duroquinone or coenzyme Q10 and is purchased from Shanghai Bidepharmatech Co., Ltd.

In the present invention, usage amounts of the aromatic diketone substance serving as a raw material and the substance having oxidability may be in any ratio. For example, the molar ratio is 1: 1, 0.01: 1, or 1: 0.01. The usage amounts of the aromatic diketone and the substance having oxidability are not 0.

When the aromatic diketone substance serving as a raw material reacts under the action of the substance having oxidability, there is no special requirement for the reaction temperature, as long as the aromatic diketone substance can react. For example, the reaction temperature is from room temperature to 150°C.

After stopping the reaction, post-processing is performed. The post-processing is performed by a conventional post-processing method, such as filtering and dialyzing, or filtering, washing, and drying, or precipitating in a precipitant.

Example 1 The preparation of n-type conjugated polymer NCPO

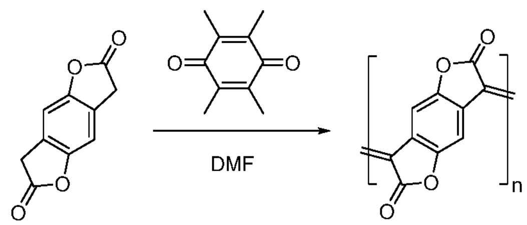


3,7-dihydrobenzo[1,2-b:4,5-b']difuran-2,6-dione (1 mmol) and duroquinone (1 mmol) were dissolved in 2 mL of DMF, and after vacuum degassing, nitrogen was filled for protection. Under the nitrogen atmosphere, an obtained solution was stirred for reaction at 100°C for 4 h. Then, a prepared crude product was diluted with DMF to about 10 mg/mL, and filtered by using a polytetrafluoroethylene filter head with a pore size of 0.45 μm. An obtained filtrate was concentrated by using a rotary evaporator, and dialysed in a DMF solution by using a dialysis bag (molecular weight cut-off = 10 kDa) for 3 days to prepare a product NCPO. The prepared NCPO was tested by gel permeation chromatography with the mobile phase of DMF,the results of test showed that the molecular weight Mn was 14 kDa and PDI was 4.02.

Example 2 The preparation of n-type conjugated polymer NCPO

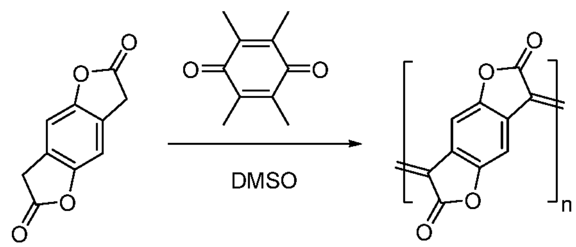


3,7-dihydrobenzo[1,2-b:4,5-b']difuran-2,6-dione (1 mmol) and duroquinone (1 mmol) were dissolved in 2 mL of DMSO, and after vacuum degassing, nitrogen was filled for protection. Under the nitrogen atmosphere, an obtained solution was stirred for reaction at 100°C for 1 h. Then, a prepared crude product was diluted with DMSO to about 10 mg/mL, and filtered by using a polytetrafluoroethylene filter head with a pore size of 0.45 μm. An obtained filtrate was concentrated by using a rotary evaporator, and dialysed in a DMSO solution by using a dialysis bag (molecular weight cut-off = 15 kDa) for 3 days to prepare a product NCPO. The prepared NCPO was tested by gel permeation chromatography with the mobile phase of DMSO, and the results of the test showed that the molecular weight Mn was 220 kDa and PDI was 2.02.

Example 3 The preparation of n-type conjugated polymer NCPO

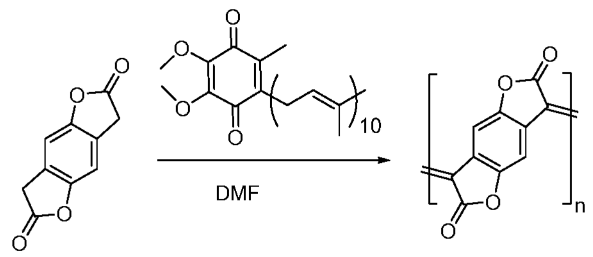


3,7-dihydrobenzo[1,2-b:4,5-b']difuran-2,6-dione (1 mmol) and coenzyme Q10 (1 mmol) were dissolved in 2 mL of DMF, and after vacuum degassing, nitrogen was filled for protection. Under the nitrogen atmosphere, an obtained solution was stirred for reaction at 100°C for 4 h. Then, a prepared crude product was diluted with DMF to about 10 mg/mL, and filtered by using a polytetrafluoroethylene filter head with a pore size of 0.45 μm. An obtained filtrate was concentrated by using a rotary evaporator, and dialysed in a DMF solution by using a dialysis bag (molecular weight cut-off = 10 kDa) for 3 days to prepare a product NCPO. The prepared NCPO was tested by gel permeation chromatography with the mobile phase of DMF, and the results of test showed that the molecular weight Mn was 21 kDa and PDI was 1.98.

Example 4 The preparation of n-type conjugated polymer NCPO

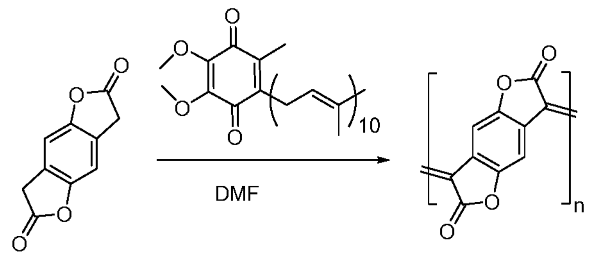


3,7-dihydrobenzo[1,2-b:4,5-b']difuran-2,6-dione (1 mmol) and coenzyme Q10 (10 mmol) were dissolved in 4 mL of DMF, and after vacuum degassing, nitrogen was filled for protection. Under the nitrogen atmosphere, an obtained solution was stirred for reaction at 100°C for 2 h. Then, a prepared crude product was diluted with DMF to about 15 mg/mL, and filtered by using a polytetrafluoroethylene filter head with a pore size of 0.45 μm. An obtained filtrate was concentrated by using a rotary evaporator, and dialysed in a DMF solution by using a dialysis bag (molecular weight cut-off = 10 kDa) for 7 days to prepare a product NCPO. The prepared NCPO was tested by gel permeation chromatography with the mobile phase of DMF, and the results of test showed that the molecular weight Mn was 76 kDa and PDI was 1.72.

Example 5 The preparation of n-type conjugated polymer NCPO

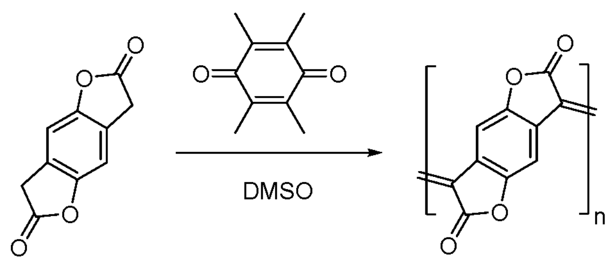


3,7-dihydrobenzo[1,2-b:4,5-b']difuran-2,6-dione (1 mmol) and duroquinone (0.1 mmol) serving as an oxidant were dissolved in 2 mL of DMSO. An obtained solution was stirred for reaction at 100°C for 24 h. Then, a prepared crude

product was diluted with DMSO to about 10 mg/mL, and filtered by using a polytetrafluoroethylene filter head with a pore size of 0.45 μm. An obtained filtrate was concentrated by using a rotary evaporator, and dialysed in a DMSO solution by using a dialysis bag (molecular weight cut-off = 15 kDa) for 3 days to prepare a product NCPO. The prepared NCPO was tested by gel permeation chromatography with the mobile phase of DMSO, and the results of test showed that the molecular weight Mn was 23 kDa and PDI was 1.20.

Example 6 The preparation of n-type conjugated polymer NCPS

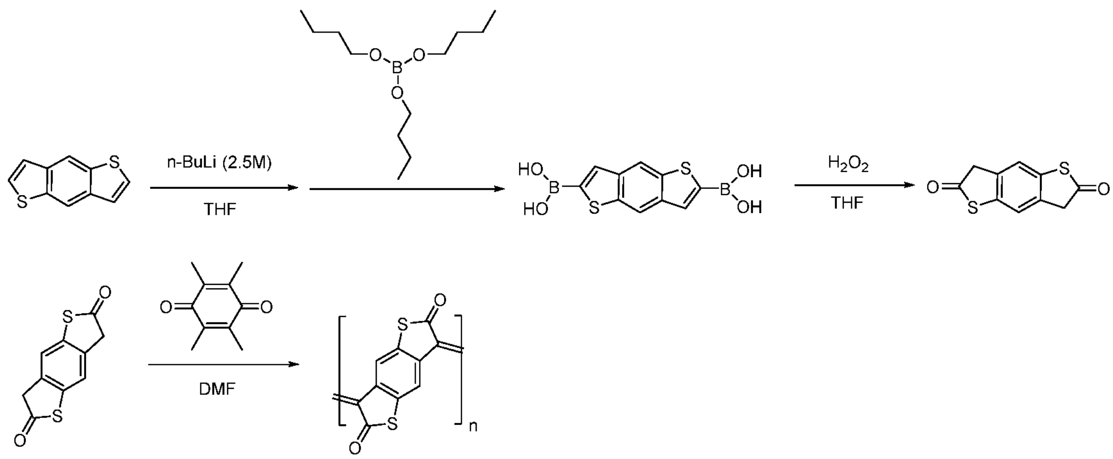


(1) Benzodithiophene (23 mmol) was dissolved in absolute tetrahydrofuran (300 mL), an obtained solution was cooled down to -78°C, n-butyllithium (a 2.5 M n-hexane solution, 25.8 mL, 64.4 mmol) was slowly added within 90 min, an obtained solution was stirred at -78°C for 3 h and heated to 0°C, tributy borate (60 mmol) was added, and an obtained solution was stirred for 1 h, gradually heated to room temperature (25°C), and stirred for 8 h. The solution was concentrated by using a rotary evaporator to 200 mL, 200 mL of 0.5 M hydrochloric acid was added, and an obtained solution was filtered to prepare a crude product. The crude product was precipitated in tetrahydrofuran/n-hexane, and an obtained precipitate was washed with ice toluene and dried to prepare a solid (with a yield of 61%) for the next reaction.

(2) The solid (7.5 mmol) obtained in the previous step was dissolved in tetrahydrofuran (100 mL), 2.5 mL of hydrogen peroxide aqueous solution (30 wt%) was added at 0°C, and an obtained solution was stirred at room temperature for 6 h. The solvent was removed by using a rotary evaporator, an obtained product was purified by using a silica gel filled chromatographic column (200-300 meshes) and using ethyl acetate: petroleum ether = 4: 1 as an eluent, and a prepared solid was washed with n-hexane and methanol to prepare 3,7-dihydrobenzo[1,2-b:4,5-b']dithiophene-2,6-dione (with a yield of 53%). $^1$H NMR ($CDCl_3$, 500 MHz) δppm: 7.31 (s, 2H), 3.98 (s, 4H).

(3) 3,7-dihydrobenzo[1,2-b:4,5-b']dithiophene-2,6-dione (1 mmol) and duroquinone (1 mmol) were dissolved in 2 mL of DMF, and after vacuum degassing, nitrogen was filled for protection. Under the nitrogen atmosphere, an obtained solution was stirred at 120°C for 48 h, diluted to about 10 mg/mL, and filtered by using a polytetrafluoroethylene filter head with a pore size of 0.45 μm. An obtained solution was concentrated by using a rotary evaporator, and dialysed in a DMF solution by using a dialysis bag (molecular weight cut-off = 5 kDa) for 3 days, and an obtained solution was adjusted with DMF to 15 mg/mL and used directly later. The solution was tested by gel permeation chromatography with the mobile phase of DMF, and the results of test showed that the molecular weight Mn was 8.9 kDa and PDI was 1.18.

Example 7 The preparation of n-type conjugated polymer NCP

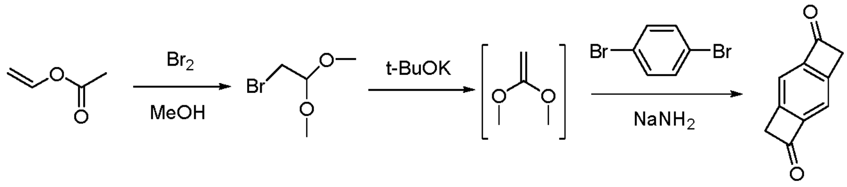


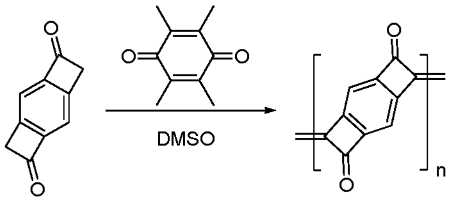


(1) 150 mL of methanol was added to a 500 mL round-bottom reaction flask, vinyl acetate (0.64 mol) and liquid bromine (0.636 mol) were slowly added at the same time under the condition of an ice salt water bath, and the temperature of the system was controlled to be below 5°C when adding dropwise vinyl acetate and liquid bromine. After vinyl acetate and liquid bromine were added, an obtained solution was gradually heated to room temperature and reacted for 12 h. The mixed solution was poured into ice water (200 mL), $NaHCO_3$ (0.67 mol) was added in batches, and an obtained solution was stirred for 30 min. After no bubble was produced, dichloromethane (3×200 mL) was added for extraction, and organic phases were combined and washed with a NaCl aqueous solution (3 × 100 mL). An obtained solution was dried with absolute magnesium sulfate, the low boiling point solvent was removed by using a rotary evaporator, and an obtained colorless liquid was used directly for the next reaction.

(2) The obtained colorless liquid was added to a 250 mL round-bottom reaction flask, absolute potassium tert-butoxide (0.64 mol) was added in batches under the condition of an ice bath, an obtained solution was heated to room temperature, stirred for 10 h, distilled, and collected to prepare a mixed solution of tert-butanol and 1,1-dimethoxyethylene, and the prepared mixed solution was used directly for the next reaction.

(3) Sodium amide (0.73 mol) was added to a 500 mL round-bottom flask, and p-dibromobenzene (0.12 mol) and 150 mL of absolute tetrahydrofuran were added. All the solution obtained in the previous step was added, and an obtained solution was stirred at 80°C for 8 h. The volatile solvent was removed by using a rotary evaporator, an obtained thick brown solid was purified by using a silica gel filled chromatographic column with an eluent of ethyl acetate: petroleum ether = 1: 5. A prepared crude product was washed with n-hexane and recrystallized with ethanol/water to prepare white solid tricyclo[6.2.0.$0^{3,6}$]dec-1,3(6),7-triene-4,9-dione (with a yield of 12%). $^1$H NMR ($CDCl_3$, 500 MHz) δppm: 7.84 (s, 2H), 4.04 (s, 4H).

(4) Tricyclo[6.2.0.$0^{3,6}$]dec-1,3(6),7-triene-4,9-dione (1 mmol) and duroquinone (1 mmol) were dissolved in 2 mL of DMSO, and after vacuum degassing, nitrogen was filled for protection. Under the nitrogen atmosphere, an obtained solution was stirred at 120°C for 48 h, diluted to about 10 mg/mL, and filtered by using a polytetrafluoroethylene filter head with a pore size of 0.45 μm. The solvent was removed from the solution by using a rotary evaporator, and a prepared solid was washed with water (10 mL), tetrahydrofuran (20 mL), and acetonitrile (20 mL) in sequence, and dried in vacuum to prepare a product NCP.

Example 8 The preparation of n-type conjugated polymer

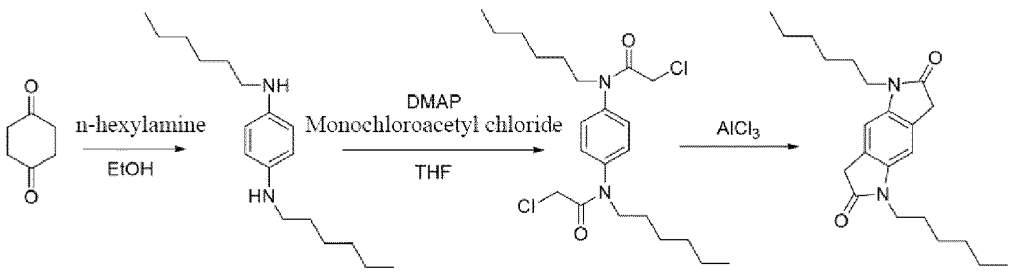


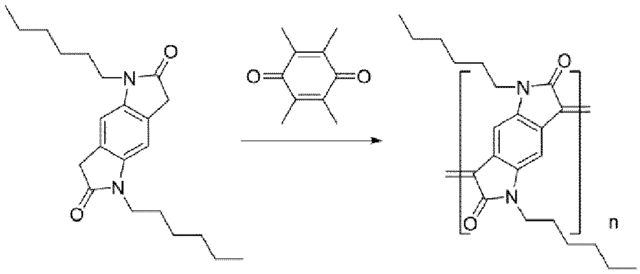

(1) 1,4-cyclohexanedione (50.0 mmol) was dissolved in 500 mL of ethanol, and n-hexylamine (100 mmol) was added to an obtained solution. An obtained mixture was stirred under air atmosphere at room temperature for 4 h. The solvent was removed through reduced pressure distillation to prepare a crude product, and the crude product was purified through column chromatography (petroleum ether: dichloromethane: ethyl acetate = 10: 10: 1) to prepare a grayish yellow solid compound (with a yield of 54.7%). $^1$H NMR ($CDCl_3$, 500 MHz) δppm: 6.54 (s, 4H), 3.15 (br, 2H), 3.03 (tr, 4H), 1.55-1.60 (m, 4H), 1.24-1.40 (m, 12H), 0.83-0.89 (m, 6H).

(2) A THF (10 mL) solution of the foregoing grayish yellow solid compound (1.19 mmol) and 4-(dimethylamino)pyridine (DMAP) (1.11 mmol) was dropwise added to a THF (5 mL) solution of chloroacetyl chloride (3.56 mmol) at 0°C. After an obtained solution was stirred for 1 h, 50 mL of water was added to quench the reaction, the solution was filtered, and an undissolved substance was collected. The collected solid was washed with water and methanol, and dried in vacuum to prepare a white solid product (with a yield of 62.7%). $^1$H NMR ($CDCl_3$, 500 MHz) δppm: 7.32 (s, 4H), 3.82 (s, 4H), 3.27 (t, 4H), 1.53-1.55 (m, 4H), 1.26-1.32 (m, 12H), 0.87-0.88 (m, 6H).

(3) Under the protection of argon, a mixture of the foregoing white solid product (11.6 mmol) and absolute aluminium chloride (81.5 mmol) was heated at 190°C for 20 min. After the mixture was cooled, ice water was added to quench the reaction, an obtained solution was filtered, and a precipitate was collected. The precipitate was washed with water and methanol, and dried to prepare a crude product, and the crude product was purified through column chromatography (dichloromethane: ethyl acetate = 10: 1) to prepare white solid 1,5-dihexyl-5,7-dihydro-1H,3H-pyrrolo[2,3-f]indole-2,6-dione (with a yield of 66.1%). $^1$H NMR ($CDCl_3$, 600 MHz) δppm: 6.78 (m, 2H), 3.68 (m, 4H), 3.55 (m, 4H), 1.61-1.69 (m, 4H), 0.98-1.48 (m, 12H), 0.88-0.89 (m, 6H).

(4) 1,5-dihexyl-5,7-dihydro-1H,3H-pyrrolo[2,3-f]indole-2,6-dione (1 mmol) and duroquinone (1 mmol) were dissolved in 2 mL of DMSO. Under the nitrogen atmosphere, an obtained solution was stirred at 120°C for 48 h, diluted to about 10 mg/mL, and filtered by using a polytetrafluoroethylene filter head with a pore size of 0.45 μm. The solvent was removed from the obtained solution by using a rotary evaporator, and an obtained solid was washed with water (10 mL), tetrahydrofuran (20 mL), and acetonitrile (20 mL) in sequence, and dried in vacuum to prepare a product NCPN (with a yield of 48%).

Example 9 The preparation of n-type conjugated polymer

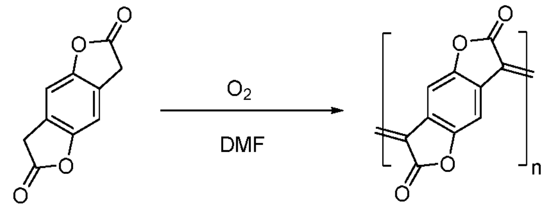


3,7-dihydrobenzo[1,2-b:4,5-b']difuran-2,6-dione (1 mmol) was dissolved in 2 mL of DMF, and air was continuously filled. An obtained solution was stirred for reaction at 100°C for 72 h. Then, a prepared crude product was diluted with DMF to about 10 mg/mL, and filtered by using a polytetrafluoroethylene filter head with a pore size of 0.45 μm, and an obtained filtrate was concentrated by using a rotary evaporator, and dialysed in a DMF solution by using a dialysis bag (molecular weight cut-off = 5 kDa) for 3 days to prepare a product NCPO. The prepared NCPO was tested by gel permeation chromatography with the mobile phase of DMF, and the results of test showed that the molecular weight Mn was 8 kDa and PDI was 1.98.

Example 10 The preparation of n-type conjugated polymer NCPO0.5-NCPS0.5

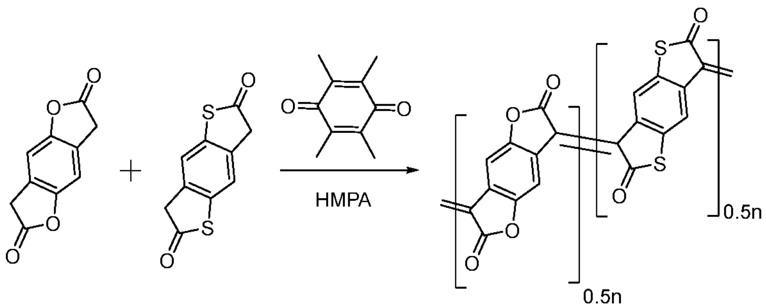


3,7-dihydrobenzo[1,2-b:4,5-b']difuran-2,6-dione (0.5 mmol) and 3,7-dihydrobenzo[1,2-b:4,5-b']dithiophene-2,6-dione (0.5 mmol) were dissolved in 2 mL of hexamethylphosphoramide (HMPA), and duroquinone (1 mmol) was added. An obtained solution was stirred for reaction at 100°C for 22 h. Then, the obtained solution was slowly dropwise added to 200 mL of dichloromethane, an obtained mixture was filtered, and a precipitate was collected, namely, product NCPO0.5-NCPS0.5. The prepared product was tested by gel permeation chromatography with the mobile phase of DMSO and the results of test showed that the molecular weight Mn was 12 kDa, and PDI was 2.01.

Example 11 The preparation of n-type conjugated polymer NCPO0.7-NCPN0.3

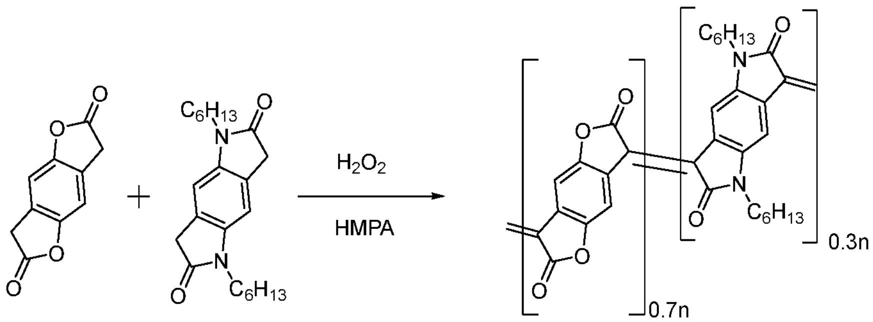


3,7-dihydrobenzo[1,2-b:4,5-b']difuran-2,6-dione (0.7 mmol) and 1,5-dihexyl-5,7-dihydro-1H,3H-pyrrolo[2,3-f]indole-2,6-dione (0.3 mmol) were dissolved in 2 mL of hexamethylphosphoramide (HMPA), and hydrogen peroxide (1 mmol, a 30 wt% aqueous solution) was added. An obtained solution was stirred for reaction at 100°C for 20 h. Then, the obtained solution was slowly dropwise added to 200 mL of dichloromethane, an obtained mixture was filtered, and

a precipitate was collected, namely, product NCPO0.7-NCPN0.3. The prepared product was tested by gel permeation chromatography with the mobile phase of DMSO, and the results of test showed that the molecular weight Mn was 2.8 kDa and PDI was 1.08.

Example 12 The preparation of n-type conjugated polymer NCPS0.5-NCPN0.5

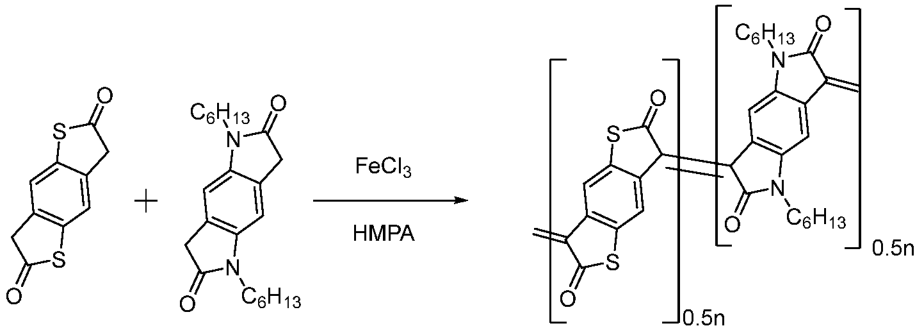


3,7-dihydrobenzo[1,2-b:4,5-b']dithiophene-2,6-dione (0.5 mmol) and 1,5-dihexyl-5,7-dihydro-1H,3H-pyrrolo[2,3-f]indole-2,6-dione (0.5 mmol) were dissolved in 2 mL of hexamethylphosphoramide (HMPA), and iron trichloride (1 mmol) was added. An obtained solution was stirred for reaction at 100°C for 72 h. Then, the obtained solution was slowly dropwise added to 200 mL of dichloromethane, an obtained mixture was filtered, and a precipitate was collected, namely, product NCPS0.5-NCPN0.5. The prepared product was tested by gel permeation chromatography with the mobile phase of DMSO, and the results of test showed that the molecular weight Mn was 3.0 kDa and PDI was 1.11.

FIG. 1(a), FIG. 1(b), and FIG. 1(b) are absorption spectrograms of the n-type conjugated polymers of Example 1, Example 6, and Example 7 in a solution or thin film state, respectively. It can be seen from the figures that the prepared n-type conjugated polymers have broad and strong absorption in the near-infrared band, which corresponds to their n-type polarons, and demonstrates the remarkable behavior of n-type conjugated polymers. It indicates that in situ doping of n-type polymers can be realized by the preparation method of the present invention without using commonly used n-type dopants to prepare conductive n-type polymers.

Test 1

The n-type conjugated polymers of Example 1 and Example 2 were adjusted to a solute concentration of 15 mg/mL with DMF (for NCPO of Example 1) and DMSO (for NCPO of Example 2), and the foregoing solutions were spin-coated to form films, and after the films were dried, the conductivity of the thin films was measured by the four-point probe method. A specific steps were as follows.

A quartz glass sheet was washed in an ultrasonic cleaner by using acetone, a special detergent for micron-level semiconductors, deionized water, and isopropanol as washing solvents in sequence, and after the washing was completed, the surface of the glass sheet was blow-dried with nitrogen, oven-dried by using an infrared lamp, and placed in a constant temperature oven for later use. Before being used, the glass sheet was bombarded with plasma in a plasma etching instrument for 10 min.

After the preparation of the glass sheet was completed, the glass sheet was placed on a heating table, heated at 110°C, transferred to a spin coater (KW-4A), and spin-coated with the foregoing prepared n-type conjugated polymer (with a mass concentration of the conjugated polymer solution of 15 mg/mL) at high speed, and the thickness of the film was measured and monitored by using a step profiler. After the formation of the film was completed, the square resistance of the film was tested by using a four-point probe conductivity tester (RTS-8 model four-point tester), and the conductivity was calculated. The results are shown in Table 1.

Table 1 The results of the conductivity of the n-type conjugated polymers of Example 1 and Example 2

| Conjugated polymer | Square resistance (Ω/□) | Thickness (nm) | Conductivity (S/cm) |
|---|---|---|---|
| Example 1 | 360 | 54 | 514 |
| Example 2 | 180 | 68 | 817 |

It can be known from Table 1 that the conjugated polymers of the present invention have high conductivity.

Test 2

The n-type conjugated polymer of Example 1 was applied in an organic photovoltaic cell as an electron transport layer, and relevant device data was tested. As a comparison example, ZnO was employed as an electron transport layer at the same time and tested under the experimental conditions of the same device structure. The organic photovoltaic cell device was prepared by a conventional method, and prepared in a glove box with levels of oxygen and water being less than 1 ppm.

A structure of the organic photovoltaic cell was as follows: ITO (100 nm)/NCPO of Example 1 (or ZnO) (50 nm)/active layer (PM6: Y6 = 1: 1.5, m/m mixed, 1 wt% diiodooctane was added) (120 nm)/molybdenum oxide (60 nm)/silver electrode (60 nm).

An effective area of a single device was 0.0516 $cm^2$.

ZnO was purchased from Sigma-Aldrich with the catalog No. 544906.

PM6 and Y6 were purchased from VAC. Structural formulas of PM6 and Y6 are as follows:

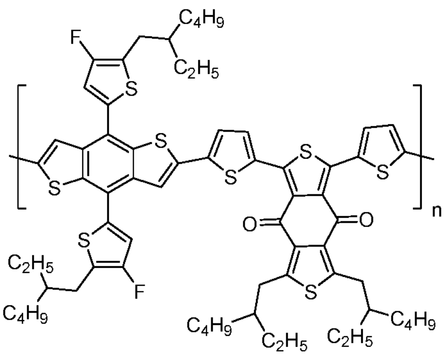

PM6

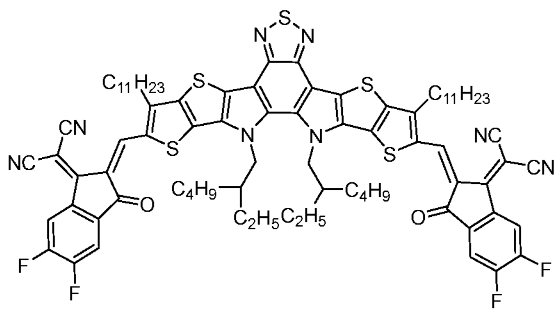

Y6

.

The energy for simulating sunlight was calibrated to 100 $mW/cm^2$ by using a silicon photoelectric diode calibrated by US National Renewable Energy Laboratory (NREL) and a KG5 filter before the test. The energy conversion efficiency of the device was measured by using a standard solar spectrum AM1.5 solar simulator (the catalog No. 91192, Oriel, USA), and current-voltage (J-V) characteristics of the photovoltaic cell device were recorded by using Keithley 2410 and Keithley 236 source-measure units, respectively.

FIG. 2 is a J-V curve chart of the organic photovoltaic cells in which ZnO and the n-type conjugated polymer of Example 1 were employed as an electron transport layer. It can be seen that the short-circuit current of the latter is obviously superior to that of the former. Obtained photoelectric conversion efficiency of the devices is shown in Table 2.

Table 2 Performance of organic photovoltaic cells based on different interface layers

| Electron transport layer of photovoltaic cell | Open-circuit voltage (V) | Short-circuit current ($mA/cm^2$) | Fill factor (%) | Photoelectric conversion efficiency (%) |
|---|---|---|---|---|
| ZnO | 0.84 | 25.51 | 71.90 | 15.38 |
| Example 1 | 0.84 | 26.12 | 74.62 | 16.30 |

It can be seen from Table 2 that two parameters, namely, the short-circuit current and the fill factor, of the organic photovoltaic cell in which NCPO of Example 1 is employed as an electron transport layer are more ideal than those of the organic photovoltaic cell in which ZnO is employed as an electron transport layer, resulting in higher final photoelectric conversion efficiency.

Test 3

A thermoelectric test was performed on NCPO of Example 1 and Example 2.

The thermoelectric performance of a material is often described by a thermoelectric figure of merit (ZT), which is specifically calculated by using the following formula:

$$ZT=S^2\sigma T/\kappa,$$

where, S denotes a Seebeck coefficient, $\sigma$ denotes the conductivity, $\kappa$ denotes thermal conductivity, and T denotes the temperature of a device during operation. The thermal conductivity of an organic material is much lower than that of an inorganic material. Therefore, the thermoelectric performance of an organic material is often described by a power factor (PF), which is calculated by using the following formula: $PF=S^2\sigma$.

In this test, the n-type conjugated polymers of Example 1 and Example 2 were taken as examples, and used to prepare a thin film on a glass substrate by drop-casting. The conductivity and the Seebeck coefficient of the films were tested to characterize the thermoelectric performance of the film. The conductivity was tested by the four-point probe method.

The glass substrate was washed with deionized water and isopropanol in sequence, and the surface was blow-dried with nitrogen. The glass was bombarded with plasma in a plasma etching instrument for 5 min. Then, different n-type conjugated polymer solutions were drop-coated on the substrates, the substrates were annealed at 80°C for 15 min, and the conductivity was measured. Conductivity test results are shown in Table 3.

Table 3 Conductivity of thin films prepared by drop-casting

| Conjugated polymer | Example 1 | Example 2 |
|---|---|---|
| Conductivity (S/cm) | 720 | 1060 |

A processing method of a device for testing the Seebeck coefficient was similar to the processing method of the device for testing the conductivity, glass was employed as a substrate, and a gold electrode was prepared by photoetching and used as a test electrode. The glass substrate was washed with deionized water and isopropanol in sequence, and the surface was blow-dried with nitrogen. The glass was bombarded with plasma in a plasma etching instrument for 5 min. Then, different n-type conjugated polymer solutions were drop-coated on the substrates, and the substrates were annealed at 80°C for 15 min.

Two ends of the device were placed in a temperature gradient field. FIG. 3(a) is a thermal voltage difference change trend diagram of the n-type conjugated polymer of Example 2, and FIG. 3(b) is a fitted change trend diagram of the thermal voltage difference with temperature of the n-type conjugated polymer of Example 2. A corresponding thermoelectromotive force was measured by changing the temperature difference between two ends of the device, and then the Seebeck coefficient was measured. It can be seen from FIG. 3(a) and FIG. 3(b) that the polymer of the example of the present invention exhibits the Seebeck effect of n-type polymers under the condition of a slight temperature difference, and produces a thermoelectromotive force exceeding 30 $\mu V/K$.

FIG. 4 is a conductivity-Seebeck coefficient-power factor performance diagram of the n-type conjugated polymers of Example 1 and Example 2. The power factor is calculated by using the following formula: $PF=S^2\sigma$. It can be obtained from the foregoing test that the n-type conjugated polymer of the present invention has the maximum power factor of 139.5 $\mu Wm^{-1}K^{-2}$ at room temperature, which is at a relatively high level among existing n-type conjugated polymers. Relevant data is shown in Table 4.

Table 4 Seebeck coefficients of thin films prepared by drop-casting

| Conjugated polymer | Example 1 | Example 2 |
|---|---|---|
| Seebeck coefficient ($\mu V/K$) | -31.48±0.16 | -36.28±1.88 |
| Power factor ($\mu Wm^{-1}K^{-2}$) | 77.5 | 139.5 |

Test 4

Application of the polymer of the present invention in electromagnetic shielding was tested. A dielectric constant and the electromagnetic shielding performance of NCPO of Example 2 were tested by a coaxial method and a waveguide

method in a band of 1-18 GHz. Experimental sample powder of NCPO of Example 2 was mixed with paraffin in a proportion by weight of 50%, and ground to prepare a powder material, and the powder material was pressed into a coaxial ring with an outer diameter of 7 mm, an inner diameter of 3.04 mm, and a thickness of 2 mm by using a mold. The sample was placed in a waveguide tube. A DR-S01 vector network analyzer was employed as a host machine. An S parameter of the sample was tested by using the network analyzer, and the reflectance and absorptance of the thin film sample were calculated based on the S parameter with reference to the paper (Review of Scientific Instruments. 2003, 74, 1098-1102), and then the electromagnetic shielding performance of the thin film sample was determined.

The electromagnetic shielding efficiency and absorption ratio of the powder material of NCPO of Example 2 are shown in FIG. 5(a). The powder material can shield more than 96% of electromagnetic waves in the broadband range of 1-18 GHz, and about 40% of electromagnetic waves are absorbed by the powder material. FIG. 5(b) is an electromagnetic shielding efficiency diagram of the polymer of Example 2 at different frequencies. FIG. 5(b) shows that the shielding effectiveness of the powder material on electromagnetic waves exceeds 20 dB, and the powder material exhibits an excellent shielding effect on broadband electromagnetic waves. The absorption shielding effectiveness corresponding to absorption of electromagnetic waves by the material exceeds 15 dB, and the average reflection shielding effectiveness corresponding to reflection of the surface of the sample is 4.2 dB. Therefore, NCPO can realize excellent electromagnetic shielding in a broadband, and thus has good application potential in electromagnetic shielding applications.

The foregoing tests indicate that the n-type conjugated polymer of the present invention can be widely applied in various organic photoelectric devices.

## Claims

1. A preparation method for an n-type conjugated polymer, comprising the following steps:

   using a solvent as a reaction medium, a reaction monomer reacting under the action of a substance having oxidability to obtain an n-type conjugated polymer,
   the reaction monomer being

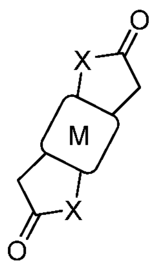

   and/or

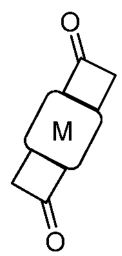

   , and/or a corresponding enol-type transformation form thereof;
   the corresponding enol-type transformation form being selected from one or more of the following structures:

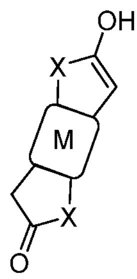 , 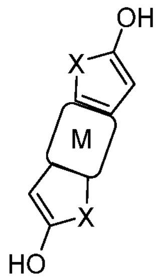 , 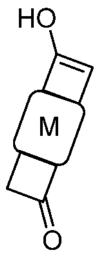 ,

and

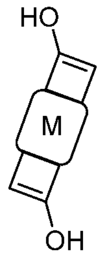 ;

the n-type conjugated polymer being a homopolymer or copolymer, comprising one or more polymerization units, the polymerization unit being a structure represented by formula I and/or a structure represented by formula II, or a corresponding enol-type transformation form thereof:

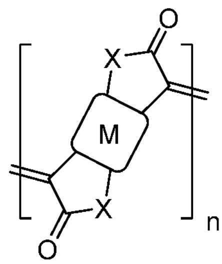
formula I

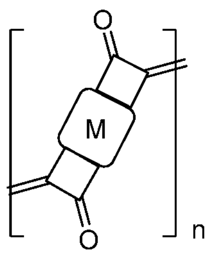
formula II;

in the polymerization unit, the corresponding enol-type transformation form being selected from:

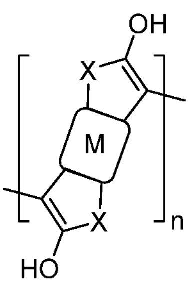
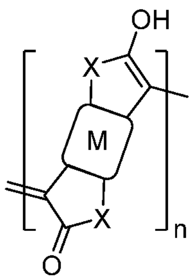
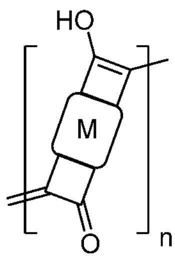
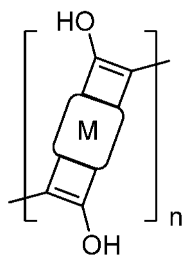
;

in the reaction monomer or polymerization unit, each X being independently selected from O, S, Se, Te or N-$R_1$, and $R_1$ being selected from one or more of a hydrogen atom, alkyl, alkylene, an alkyl derivative, and an alkylene derivative;
in the reaction monomer or polymerization unit, each M being independently a conjugated part selected from one of an aromatic ring, a heteroaromatic ring, a fused aromatic ring or a fused heteroaromatic ring; and
in formula I, formula II or the corresponding enol-type transformation form, n being a positive integer.

**2.** The preparation method of the n-type conjugated polymer according to claim 1, wherein

in $R_1$, the alkyl derivative refers to that one or more carbons on alkyl are substituted with one or more of an oxygen atom, amino, sulfonyl, carbonyl, aryl, alkenyl, alkynyl, ester, cyano, and nitro;
and/or
one or more hydrogens on the alkyl derivative are substituted with one or more of a halogen, hydroxyl, amino, carboxyl, cyano, nitro, aryl, alkenyl, and alkyne;
M is independently selected from the following structures:

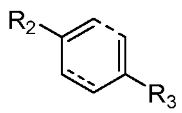 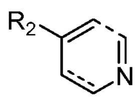 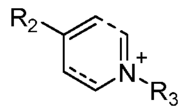 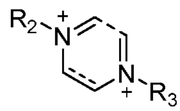

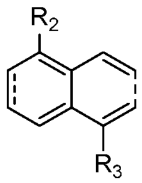 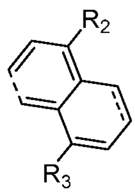 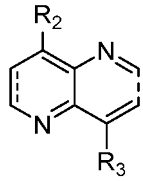 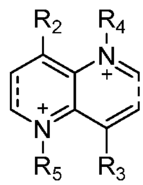

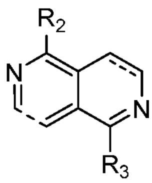 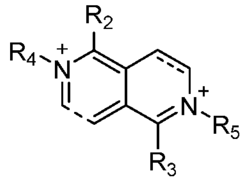 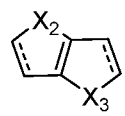 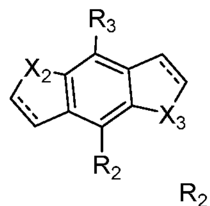

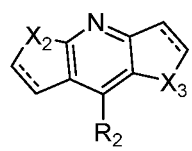 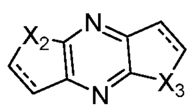 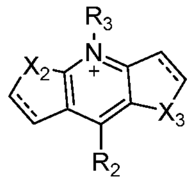 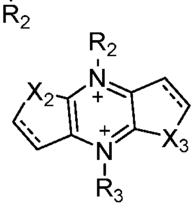

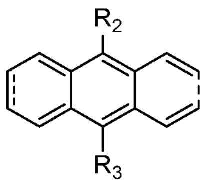 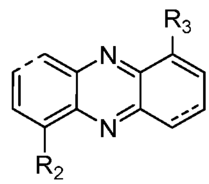 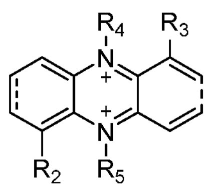 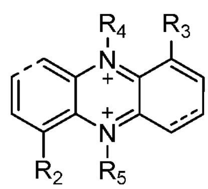

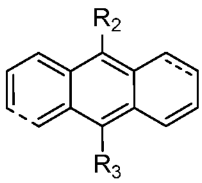 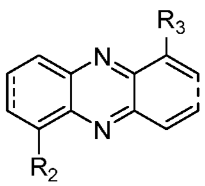 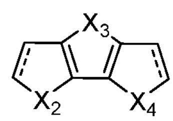 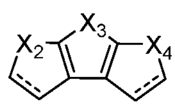 ;

$X_2$ to $X_4$ are independently selected from O, S, Se, Te or N-$R_1$; and $R_1$ in N-Ri is selected from one or more of a hydrogen atom, alkyl, alkylene, an alkyl derivative, and an alkylene derivative;
$R_2$ to $R_5$ are independently selected from one or more of a hydrogen atom, hydroxyl, nitro, a halogen, cyano, nitro, alkyl, and an alkyl derivative;
in the foregoing structure of M, a dotted line --- in the aromatic ring indicates mutual fusion of the aromatic ring and an adjacent five-membered ring or four-membered ring at this place, namely, a shared ring edge;
the substance having oxidability is selected from one or more of an organic substance having oxidability and

an inorganic substance having oxidability;
the solvent is selected from a solvent 1, a solvent 2, or a mixture of the solvent 1 and the solvent 2;
the solvent 1 is selected from one or more of water, a nitrile solvent, an aromatic solvent, an alicyclic hydrocarbon solvent, an alicyclic hydrocarbon solvent, a halogenated hydrocarbon solvent, an alcohol solvent, an ether solvent, an ester solvent, a sulfone solvent, a ketone solvent, an amide solvent; and
the solvent 2 is a deuterated solvent of the solvent 1.

**3.** The preparation method of the n-type conjugated polymer according to claim 2, wherein

the substance having oxidability is more than one of oxygen, a peroxide, a metal halide, a persulfate, a perborate, a hypohalite, a chlorite, a bromite, a quinone compound, and a perbenzoate compound;
the solvent 1 is more than one of tetrahydrofuran, methyltetrahydrofuran, dichloromethane, chloroform, ethyl acetate, propyl acetate, butyl acetate, ethyl propionate, propyl propionate, butyl propionate, methyl methacrylate, ethyl methacrylate, propyl methacrylate, toluene, xylene, trimethylbenzene, chlorobenzene, dichlorobenzene, trichlorobenzene, methanol, ethanol, propanol, ethylene glycol, isobutanol, propylene glycol, acetonitrile, formic acid, acetic acid, propionic acid, trifluoroacetic acid, dimethylformamide, dimethylacetamide, dimethyl sulfoxide, dimethylacetamide, acetone, butanone, cyclohexanone, methyl butanone, methyl ether, diethyl ether, propyl ether, pyridine, phenol, N-methylpyrrolidone, ethylene glycol monomethyl ether, triethylene glycol monomethyl ether, triethylamine, tetramethylethylenediamine, trioctylamine, aniline, and hexamethylphosphoramide; and
the solvent 2 is a deuterated solvent corresponding to the solvent 1.

**4.** The preparation method of the n-type conjugated polymer according to claim 1, wherein

a structure of the n-type conjugated polymer is formula I, formula II, a structure composed of formula I and formula II, a structure composed of formula I and formula I, a structure composed of formula II and formula II, an enol-type transformation form corresponding to formula I, an enol-type transformation form corresponding to formula II, a structure composed of formula I and the corresponding enol-type transformation form thereof, a structure composed of formula II and the corresponding enol-type transformation form thereof, or a structure composed of formula I, formula II, and the corresponding enol-type transformation forms; in the structure composed of formula I and formula I, formula I is different from formula I, in the structure composed of formula II and formula II, formula II is different from formula II; and the structure composed of formula I and formula II contains one or more formulae I, and contains one or more formulae II, formulae I are the same or different, and formulae II are the same or different;
in a case that the n-type conjugated polymer is a homopolymer, the reaction monomers are compounds with the same structure and/or the corresponding enol-type transformation form thereof; and
in a case that the n-type conjugated polymer is a copolymer, the reaction monomers are compounds with different structures or compounds with different structures and corresponding enol-type transformation forms thereof.

**5.** The preparation method of the n-type conjugated polymer according to claim 4, wherein

in a case that the n-type conjugated polymer is a homopolymer, the structure of the n-type conjugated polymer is selected from one of the following structures, or corresponding enol-type transformation forms thereof:

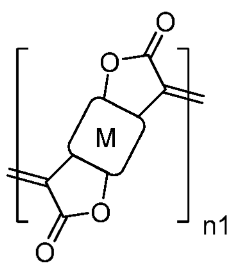 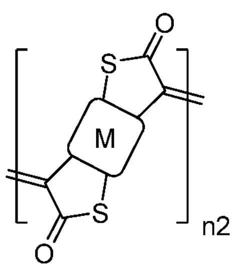 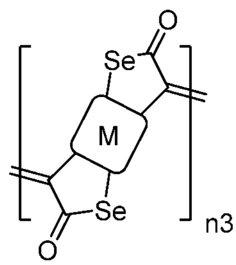

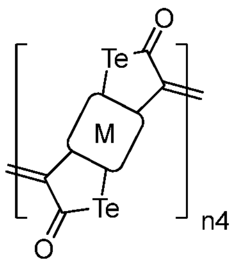
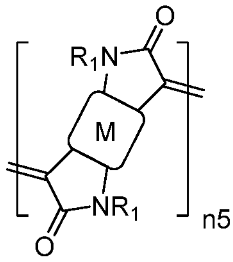
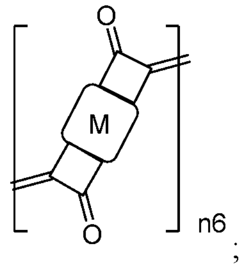

the corresponding enol-type transformation forms are as follows:

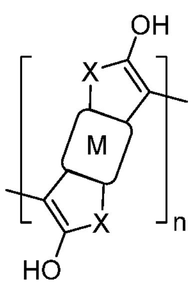
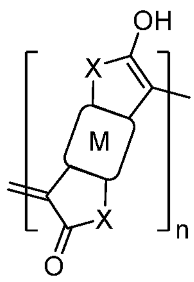
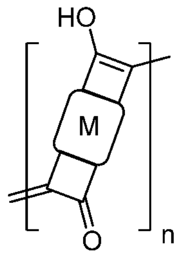
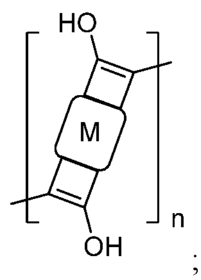

X is correspondingly selected from O, S, Se, Te or N-$R_1$; and in the corresponding enol-type transformation form, n is a positive integer;

in a case that the n-type conjugated polymer is a copolymer, the n-type conjugated polymer comprises at least two polymerization units;

a structure of each polymerization unit is independently selected from the following structures or corresponding enol-type transformation forms thereof:

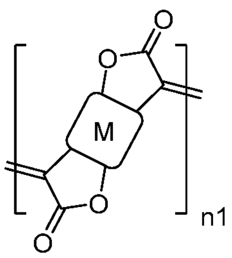
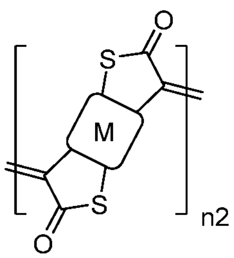
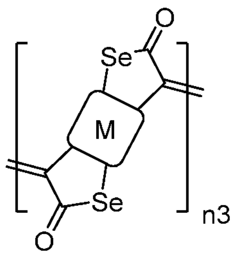

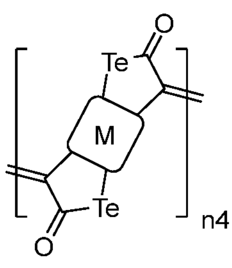
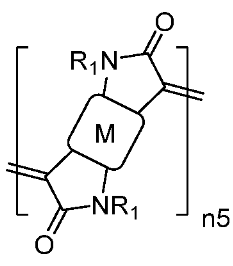
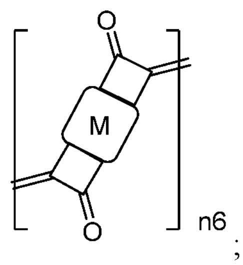

the corresponding enol-type transformation forms are as follows:

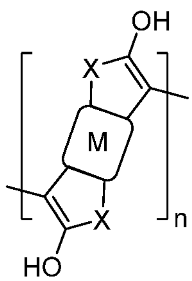 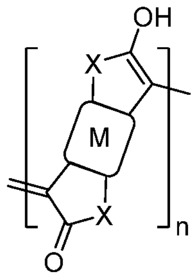 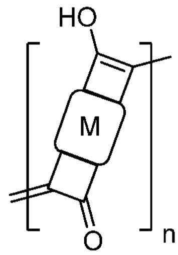 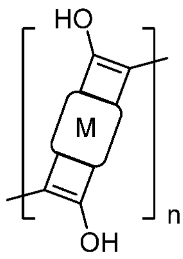 ;

X is correspondingly selected from O, S, Se, Te or N-$R_1$; and in the corresponding enol-type transformation form, n is a positive integer;
wherein
in the n-type conjugated polymer, $R_1$ is selected from one or more of a hydrogen atom, alkyl, alkylene, an alkyl derivative, and an alkylene derivative;
one or more carbons on the alkyl derivative or alkylene derivative are substituted with one or more of an oxygen atom, amino, sulfonyl, carbonyl, aryl, alkenyl, alkynyl, ester, cyano, and nitro;
and/or
one or more hydrogens on the alkyl derivative or alkylene derivative are substituted with one or more of a halogen, hydroxyl, amino, carboxyl, cyano, nitro, aryl, alkenyl, and alkyne;
n1 to n6 are independently a positive integer; and
a structure of M is selected from one of an aromatic ring, a heteroaromatic ring, a fused aromatic ring, and a fused heteroaromatic ring.

**6.** An n-type conjugated polymer prepared by the preparation method according to any one of claims 1 to 5, wherein the n-type conjugated polymer is a homopolymer or copolymer;

the n-type conjugated polymer comprises one or more polymerization units, and the polymerization unit is a structure represented by formula I and/or a structure represented by formula II, and/or a corresponding enol-type transformation form thereof;

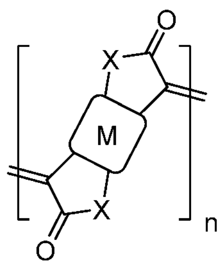 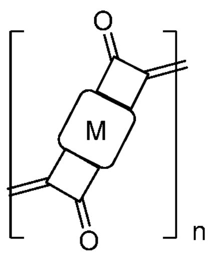

formula I formula II;

in formula I, each X is independently selected from O, S, Se, Te or N-$R_1$; and $R_1$ is selected from one or more of a hydrogen atom, alkyl, alkylene, an alkyl derivative, and an alkylene derivative;
in formula I or formula II, each M is independently a conjugated part selected from one of an aromatic ring, a heteroaromatic ring, a fused aromatic ring, and a fused heteroaromatic ring;
in formula I or formula II, n is a positive integer;
the corresponding enol-type transformation type refers to an enol-type transformation form corresponding to the structure represented by formula I or an enol-type transformation form corresponding to the structure represented by formula II; and
the corresponding enol-type transformation form is selected from more than one of the following structures:

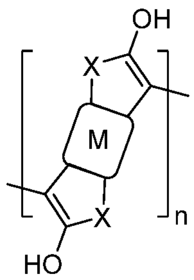 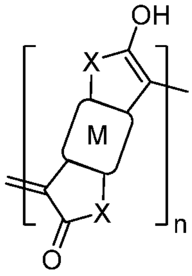 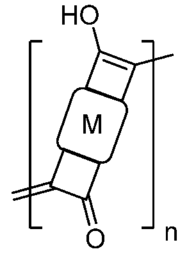 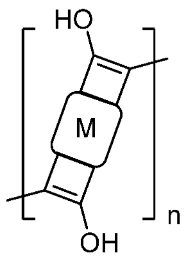

; and n is a positive integer.

7. A use of the n-type conjugated polymer according to claim 6 in an organic photoelectric device, wherein the n-type conjugated polymer is applied to an electron transport layer, a thermoelectric material and/or an electromagnetic shielding material.

8. An electron transport layer, comprising an n-type conjugated polymer,

the n-type conjugated polymer being a homopolymer or copolymer, the n-type conjugated polymer comprising one or more polymerization units, and the polymerization unit being a structure represented by formula I and/or a structure represented by formula II, and/or a corresponding enol-type transformation form thereof:

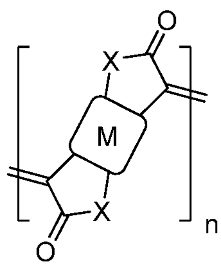 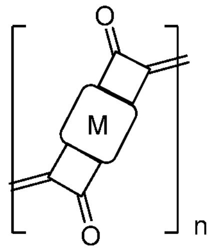

formula I formula II;

in formula I, X being independently selected from O, S, Se, Te or N-$R_1$; and $R_1$ being selected from one or more of a hydrogen atom, alkyl, alkylene, an alkyl derivative, and an alkylene derivative
in formula I or formula II, each M being independently a conjugated part selected from one of an aromatic ring, a heteroaromatic ring, a fused aromatic ring, and a fused heteroaromatic ring;
in formula I or formula II, n being a positive integer;
the corresponding enol-type transformation type referring to an enol-type transformation form corresponding to the structure represented by formula I or an enol-type transformation form corresponding to the structure represented by formula II; and
the corresponding enol-type transformation form being selected from more than one of the following structures:

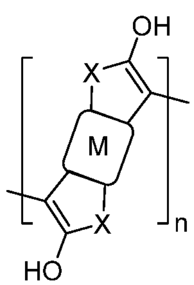 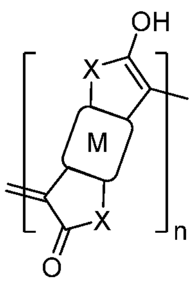 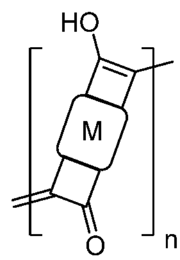 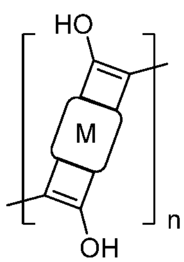

; and n being a positive integer.

9. A thermoelectric material, comprising an n-type conjugated polymer,

the n-type conjugated polymer being a homopolymer or copolymer, the n-type conjugated polymer comprising one or more polymerization units, and the polymerization unit being a structure represented by formula I and/or a structure represented by formula II, and/or a corresponding enol-type transformation form thereof:

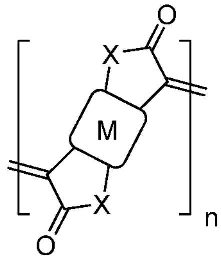

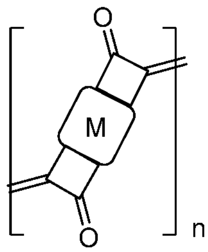

formula I formula II;

in formula I, X being independently selected from O, S, Se, Te or N-$R_1$; and $R_1$ being selected from one or more of a hydrogen atom, alkyl, alkylene, an alkyl derivative, and an alkylene derivative;
in formula I or formula II, each M being independently a conjugated part selected from one of an aromatic ring, a heteroaromatic ring, a fused aromatic ring, and a fused heteroaromatic ring;
in formula I or formula II, n being a positive integer;
the corresponding enol-type transformation type referring to an enol-type transformation form corresponding to the structure represented by formula I or an enol-type transformation form corresponding to the structure represented by formula II; and
the corresponding enol-type transformation form being selected from more than one of the following structures:

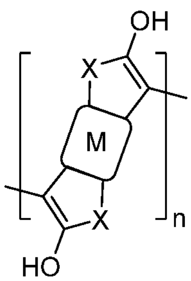

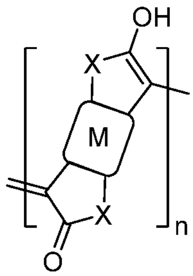

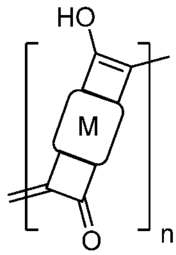

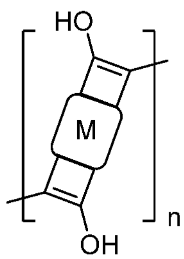

; and n being a positive integer.

**10.** An electromagnetic shielding material, comprising an n-type conjugated polymer,

the n-type conjugated polymer being a homopolymer or copolymer, the n-type conjugated polymer comprising one or more polymerization units, and the polymerization unit being a structure represented by formula I and/or a structure represented by formula II, and/or a corresponding enol-type transformation form thereof:

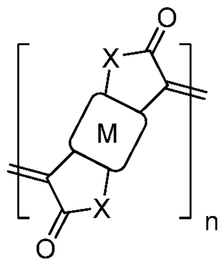

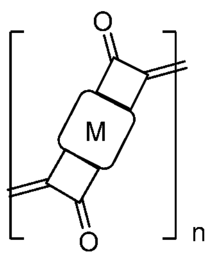

formula I formula II;

in formula I, X being independently selected from O, S, Se, Te or N-$R_1$; and $R_1$ being selected from one or more of a hydrogen atom, alkyl, alkylene, an alkyl derivative, and an alkylene derivative;

in formula I or formula II, each M being independently a conjugated part selected from one of an aromatic ring, a heteroaromatic ring, a fused aromatic ring, and a fused heteroaromatic ring;
in formula I or formula II, n being a positive integer;
the corresponding enol-type transformation type referring to an enol-type transformation form corresponding to the structure represented by formula I or an enol-type transformation form corresponding to the structure represented by formula II; and
the corresponding enol-type transformation form being selected from more than one of the following structures:

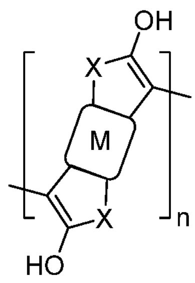
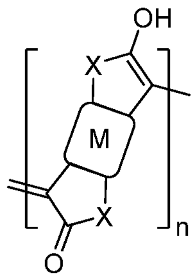
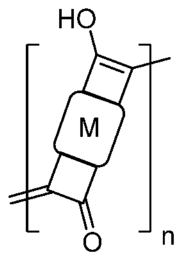
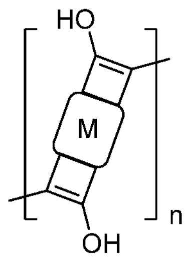

; and n being a positive integer.

**11.** A supramolecular n-type conjugated polymer, formed by self-assembly, under the intermolecular interaction, of different resonance forms that are formed by a structure represented by formula I or a structure represented by formula II and a corresponding enol-type transformation form:

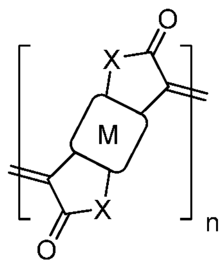
formula I

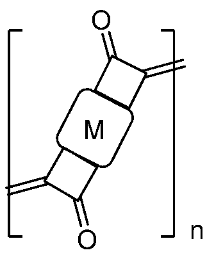
formula II;

in formula I, X being independently selected from O, S, Se, Te or N-$R_1$; and $R_1$ being selected from one or more of a hydrogen atom, alkyl, alkylene, an alkyl derivative, and an alkylene derivative;
in formula I or formula II, each M being independently a conjugated part selected from one of an aromatic ring, a heteroaromatic ring, a fused aromatic ring, and a fused heteroaromatic ring;
in formula I or formula II, n being a positive integer;
the corresponding enol-type transformation type referring to an enol-type transformation form corresponding to the structure represented by formula I or an enol-type transformation form corresponding to the structure represented by formula II;
the corresponding enol-type transformation form being selected from more than one of the following structures:

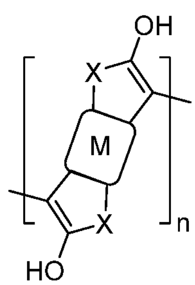
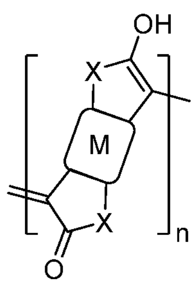
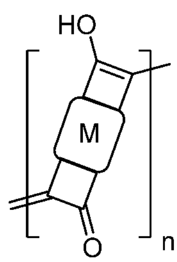
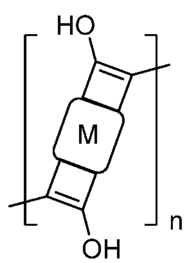

; and n being a positive integer; and
in a case that M is a benzene ring, the supramolecular n-type conjugated polymer having the following structure:

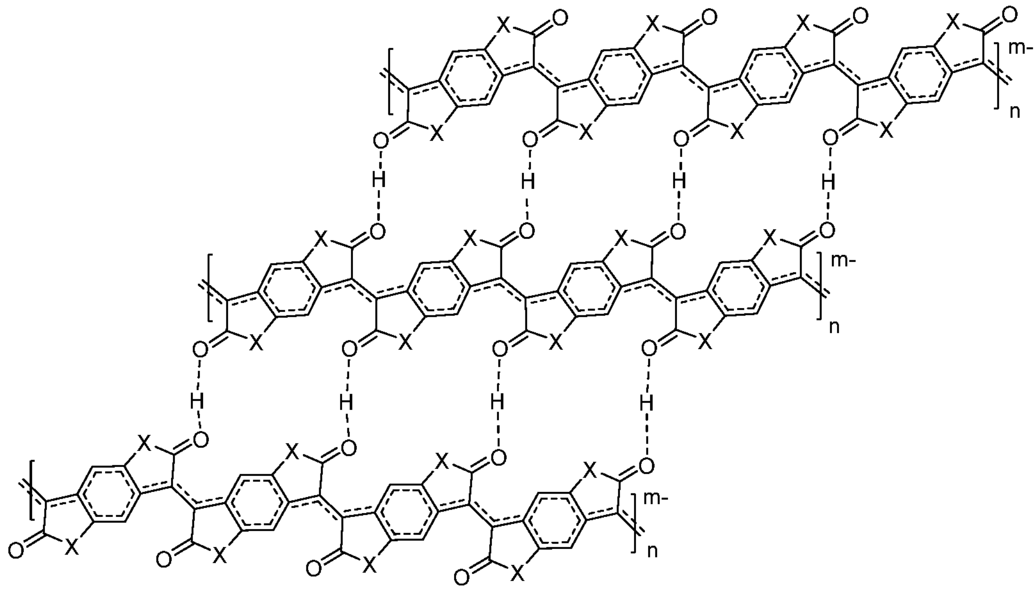

12. The supramolecular n-type conjugated polymer according to claim 11, wherein in a case that X is an oxygen atom, and M is a benzene ring structure, the supramolecular n-type conjugated polymer has the following tautomers:

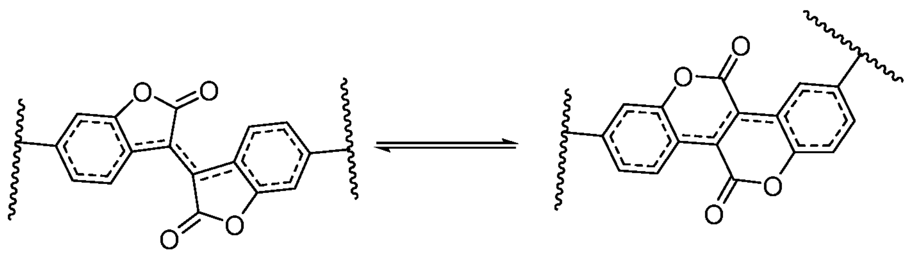

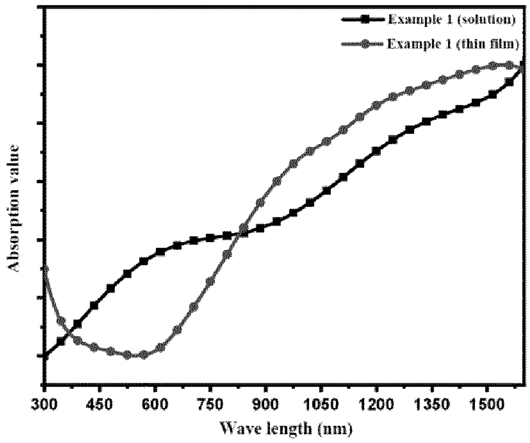


FIG. 1(a)

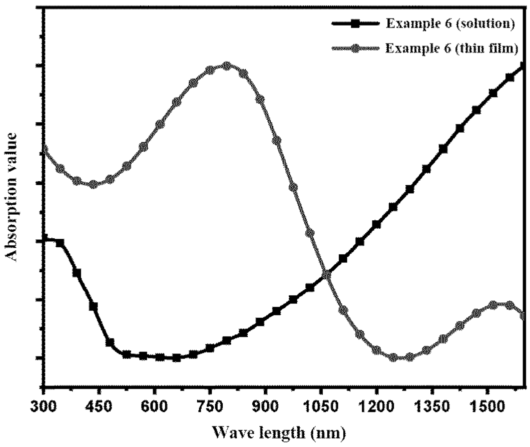


FIG. 1(b)

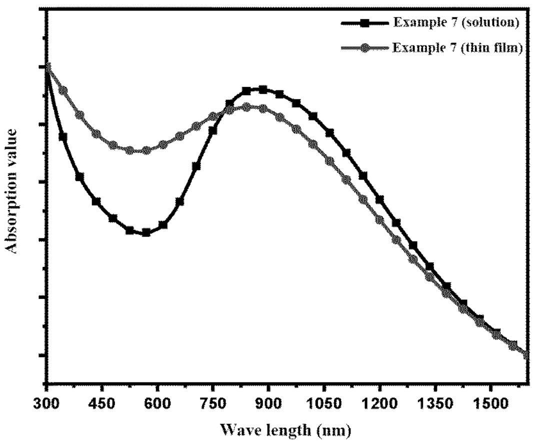


FIG. 1(c)

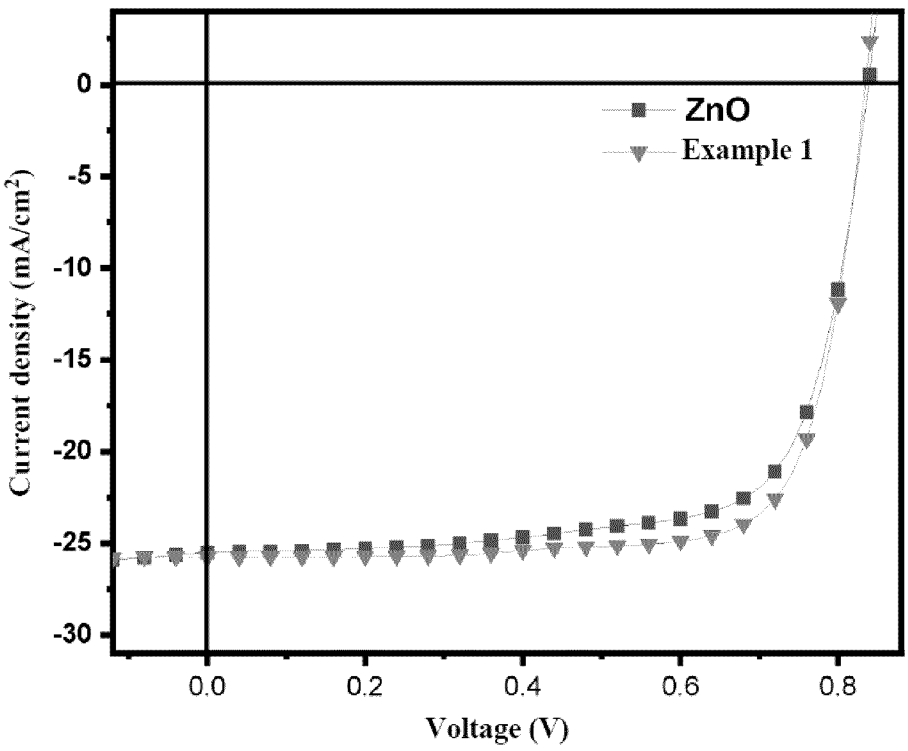
ZnO
Example 1
Current density (mA/cm2)
Voltage (V)
0
-5
-10
-15
-20
-25
-30
0.0
0.2
0.4
0.6
0.8

FIG. 2

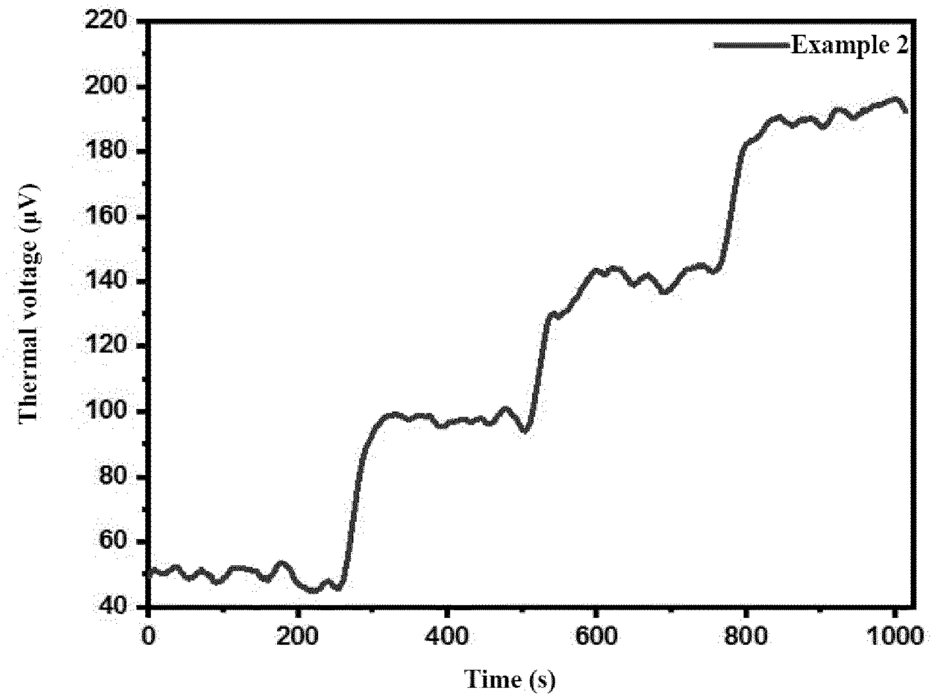
Example 2
Thermal voltage (μV)
Time (s)
220
200
180
160
140
120
100
80
60
40
0
200
400
600
800
1000

FIG. 3(a)

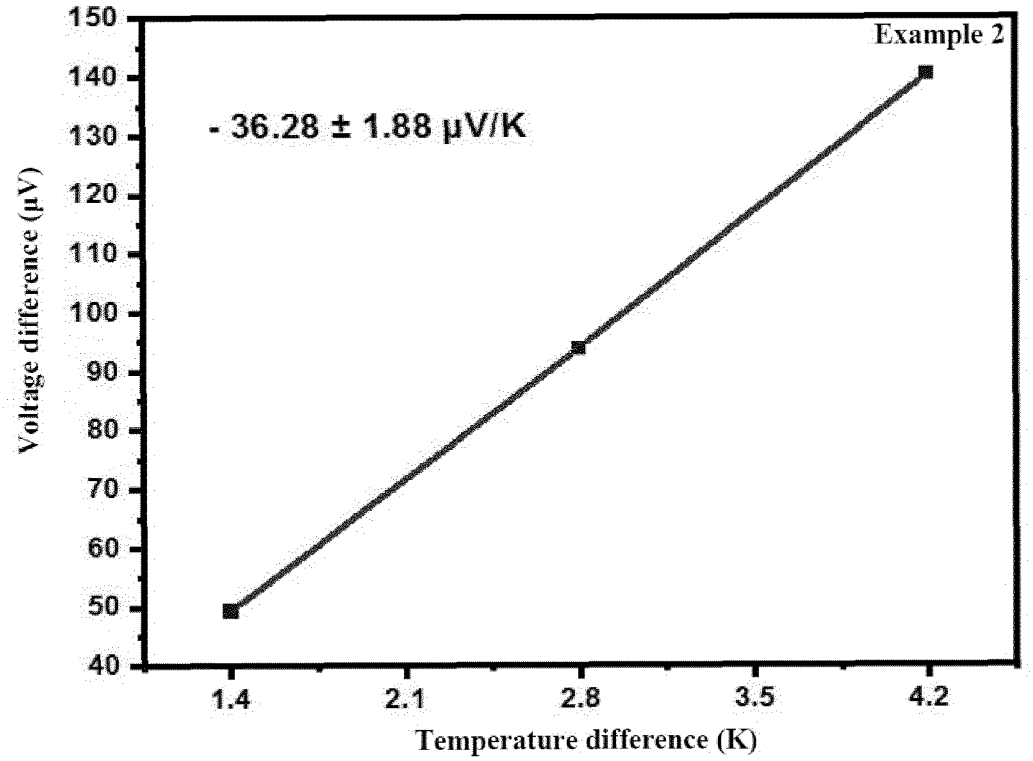
Example 2
- 36.28 ± 1.88 µV/K
Voltage difference (µV)
150
140
130
120
110
100
90
80
70
60
50
40
1.4
2.1
2.8
3.5
4.2
Temperature difference (K)

FIG. 3(b)

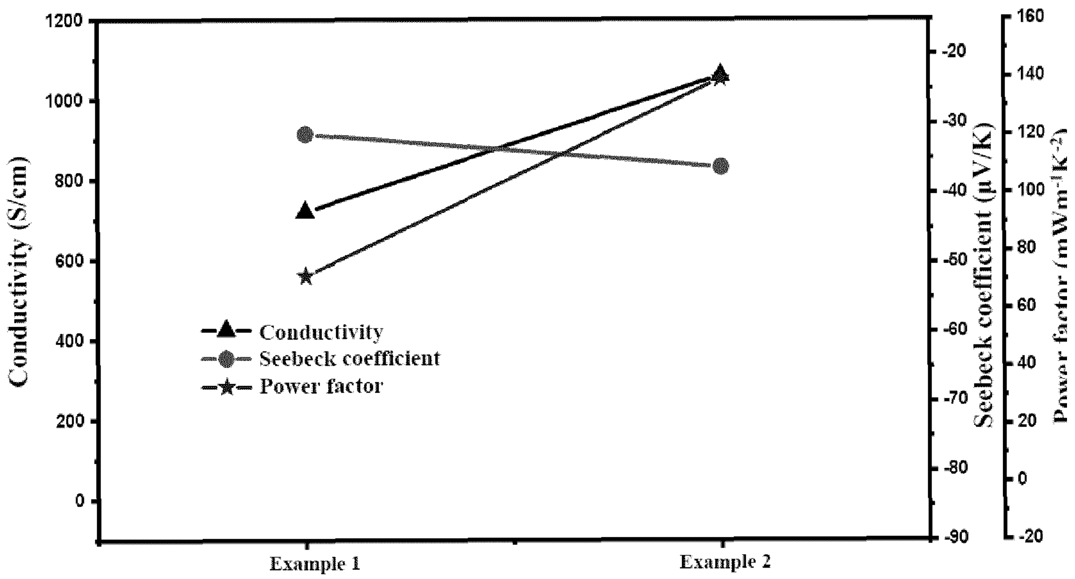
Conductivity (S/cm)
1200
1000
800
600
400
200
0
Conductivity
Seebeck coefficient
Power factor
Seebeck coefficient (µV/K)
-20
-30
-40
-50
-60
-70
-80
-90
160
140
120
100
80
60
40
20
0
-20
Example 1
Example 2

FIG. 4

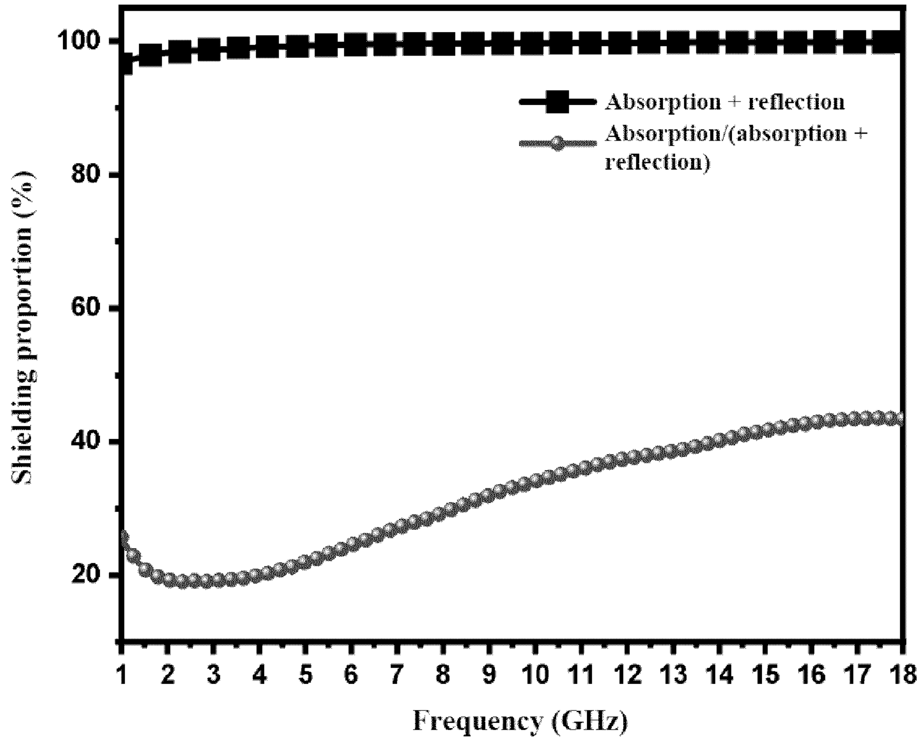
Absorption + reflection
Absorption/(absorption + reflection)
Shielding proportion (%)
100
80
60
40
20
1
2
3
4
5
6
7
8
9
10
11
12
13
14
15
16
17
18
Frequency (GHz)

FIG. 5(a)

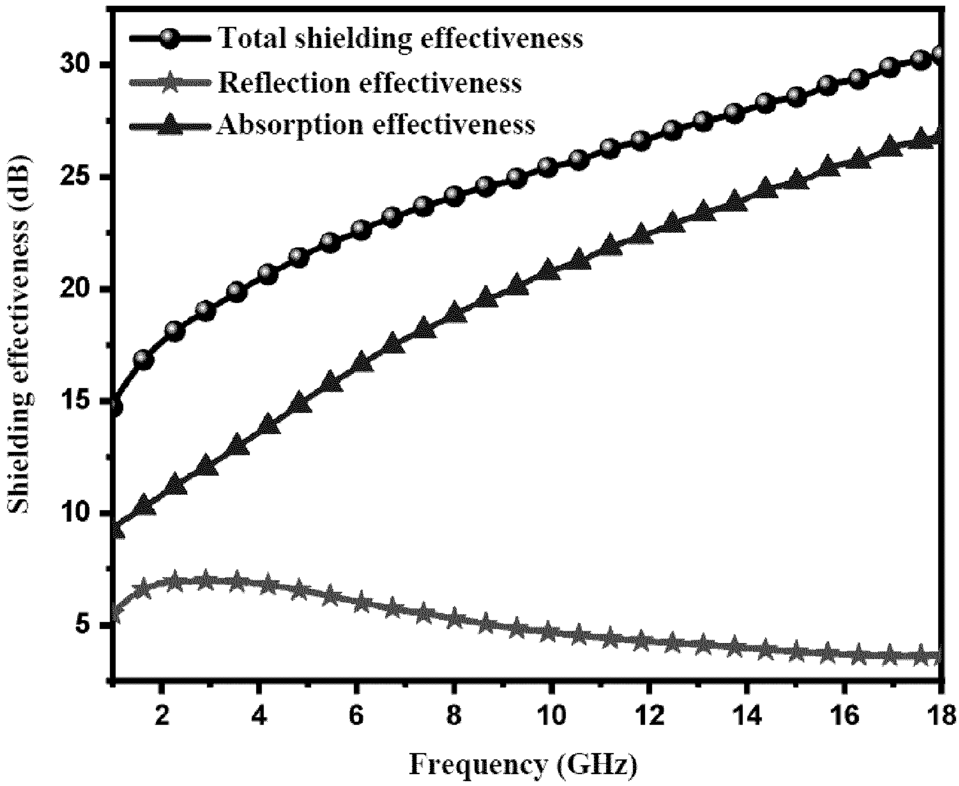
Total shielding effectiveness
Reflection effectiveness
Absorption effectiveness
Shielding effectiveness (dB)
30
25
20
15
10
5
2
4
6
8
10
12
14
16
18
Frequency (GHz)

FIG. 5(b)

**INTERNATIONAL SEARCH REPORT**

International application No.
**PCT/CN2021/124880**

**A. CLASSIFICATION OF SUBJECT MATTER**

C08G 61/12(2006.01)i; H01L 51/30(2006.01)i; H01L 51/46(2006.01)i; H01L 51/54(2006.01)i

According to International Patent Classification (IPC) or to both national classification and IPC

**B. FIELDS SEARCHED**

Minimum documentation searched (classification system followed by classification symbols)

C08G 61/-;H01L 51/-

Documentation searched other than minimum documentation to the extent that such documents are included in the fields searched

Electronic data base consulted during the international search (name of data base and, where practicable, search terms used)

CNKI; CPRSABS; EPODOC; WPI; WEB OF SCIENCE: 华南理工大学, 黄飞, 苯并, 二呋喃, 二噻吩, 二吡咯, 二环丁, 二酮, 四酮, huang fei, +benzo+, +difuran+, +dithiophene+, +dipyrrol+, +cyclobutene+, +dione, +tetrone

**C. DOCUMENTS CONSIDERED TO BE RELEVANT**

| Category* | Citation of document, with indication, where appropriate, of the relevant passages | Relevant to claim No. |
|---|---|---|
| X | CN 104812795 A (LI YUNING) 29 July 2015 (2015-07-29) claims 1-16 | 6-8, 10 |
| A | CN 104812795 A (LI YUNING) 29 July 2015 (2015-07-29) claims 1-16 | 1-5, 9, 11, 12 |
| X | CN 108699073 A (BASF SE) 23 October 2018 (2018-10-23) claims 1-20 | 6, 10-12 |
| A | CN 108699073 A (BASF SE) 23 October 2018 (2018-10-23) claims 1-20 | 1-5, 7-9 |
| X | CN 103025788 A (MERCK PATENT GMBH) 03 April 2013 (2013-04-03) claims 1-15 | 6-8, 10 |
| A | CN 103025788 A (MERCK PATENT GMBH) 03 April 2013 (2013-04-03) claims 1-15 | 1-5, 9, 11, 12 |
| X | DE 3618838 A1 (BASF AG.) 11 December 1986 (1986-12-11) claims 1-4 | 6-8, 10 |
| A | DE 3618838 A1 (BASF AG.) 11 December 1986 (1986-12-11) claims 1-4 | 1-5, 9, 11, 12 |

☑ Further documents are listed in the continuation of Box C. ☑ See patent family annex.

* Special categories of cited documents:
- "A" document defining the general state of the art which is not considered to be of particular relevance
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **16 March 2022** | **28 March 2022** |

| Name and mailing address of the ISA/CN | Authorized officer |
|---|---|
| **China National Intellectual Property Administration (ISA/CN)**<br>**No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

**INTERNATIONAL SEARCH REPORT**

International application No.
**PCT/CN2021/124880**

**C. DOCUMENTS CONSIDERED TO BE RELEVANT**

| Category* | Citation of document, with indication, where appropriate, of the relevant passages | Relevant to claim No. |
|---|---|---|
| X | ZHOU, Xu. "Balanced Ambipolar Organic Thin-Film Transistors Operated under Ambient Conditions: Role of the Donor Moiety in BDOPV-Based Conjugated Copolymers" *Chemistry of Materials,* Vol. 27, No. 5, 10 March 2015 (2015-03-10), pp. 1815-1820 | 6-8, 10 |

Form PCT/ISA/210 (second sheet) (January 2015)

**INTERNATIONAL SEARCH REPORT**
**Information on patent family members**

International application No.
**PCT/CN2021/124880**

| Patent document cited in search report | | | Publication date (day/month/year) | Patent family member(s) | | | Publication date (day/month/year) |
|---|---|---|---|---|---|---|---|
| CN | 104812795 | A | 29 July 2015 | US | 2015295179 | A1 | 15 October 2015 |
| | | | | EP | 2917261 | A1 | 16 September 2015 |
| | | | | EP | 2917261 | A4 | 16 November 2016 |
| | | | | KR | 20150082540 | A | 15 July 2015 |
| | | | | JP | 2016505516 | A | 25 February 2016 |
| | | | | WO | 2014071524 | A1 | 15 May 2014 |
| CN | 108699073 | A | 23 October 2018 | EP | 3423455 | A1 | 09 January 2019 |
| | | | | EP | 3423455 | B1 | 23 June 2021 |
| | | | | US | 2019048015 | A1 | 14 February 2019 |
| | | | | US | 10815236 | B2 | 27 October 2020 |
| | | | | KR | 20180113610 | A | 16 October 2018 |
| | | | | WO | 2017148864 | A1 | 08 September 2017 |
| | | | | TW | 201800412 | A | 01 January 2018 |
| | | | | TW | I717468 | B | 01 February 2021 |
| | | | | JP | 2019508556 | A | 28 March 2019 |
| CN | 103025788 | A | 03 April 2013 | WO | 2012003918 | A1 | 12 January 2012 |
| | | | | RU | 2013105371 | A | 20 August 2014 |
| | | | | KR | 20130100978 | A | 12 September 2013 |
| | | | | KR | 101792901 | B1 | 02 November 2017 |
| | | | | JP | 2013531106 | A | 01 August 2013 |
| | | | | JP | 5789298 | B2 | 07 October 2015 |
| | | | | EP | 2591038 | A1 | 15 May 2013 |
| | | | | EP | 2591038 | B1 | 05 November 2014 |
| | | | | SG | 186874 | A1 | 28 February 2013 |
| | | | | GB | 201300592 | D0 | 27 February 2013 |
| | | | | GB | 2494607 | A | 13 March 2013 |
| | | | | GB | 2494607 | B | 02 May 2018 |
| | | | | CN | 103025788 | B | 12 August 2015 |
| | | | | US | 2013175481 | A1 | 11 July 2013 |
| | | | | US | 9287504 | B2 | 15 March 2016 |
| | | | | DE | 112011102301 | T5 | 16 May 2013 |
| | | | | TW | 201209071 | A | 01 March 2012 |
| | | | | TW | I508992 | B | 21 November 2015 |
| DE | 3618838 | A1 | 11 December 1986 | | None | | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2014071524 A
- US 20190048015 A1
- CN 108699073


### Non-patent literature cited in the description


- Toward High Performance n-Type Thermoelectric Materials by Rational Modification of BDPPV Backbones. *J. Am. Chem. Soc.,* 2015, vol. 137, 6979
- High Conductivity and Electron-Transfer Validation in an n-Type Fluoride-Anion-Doped Polymer for Thermoelectrics in Air. *Adv. Mater.,* 2017, vol. 29, 1606928
- Rigid Coplanar Polymers for Stable n-Type Polymer Thermoelectrics. *Angew. Chem. Int. Ed,* 2019, vol. 58, 11390
- Synthese, Charakterisierung und Halbleitereigenschaften eines polymeren Indopheninhomologen. *Macro. Chem. Phys,* 1975, vol. 176, 333
- A BDOPV-Based Donor-Acceptor Polymer for High-Performance n-Type and Oxygen-Doped Ambipolar Field-Effect Transistors. *Adv. Mater,* 2013, vol. 25 (45), 6589
- *Review of Scientific Instruments,* 2003, vol. 74, 1098-1102